(19) Europäisches Patentamt
European Patent Office
Office européen des brevets

(11) **EP 4 686 763 A1**

(12) **EUROPEAN PATENT APPLICATION**

(43) Date of publication:
**04.02.2026 Bulletin 2026/06**

(21) Application number: 24306297.3

(22) Date of filing: **31.07.2024**

(51) International Patent Classification (IPC):
***C12Q 1/6883*** (2018.01)

(52) Cooperative Patent Classification (CPC):
**C12Q 1/6883;** C12Q 2600/158

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
- **Institut National de la Recherche pour l'Agriculture, l'Alimentation et l'Environnement**
  **75007 Paris (FR)**
- **Centre National de la Recherche Scientifique**
  **75016 Paris (FR)**
- **Université Clermont Auvergne**
  **63000 Clermont-Ferrand (FR)**

(72) Inventors:
- **JOUSSE, Céline**
  **63122 Saint-Genès-Champanelle (FR)**
- **FAFOURNOUX, Pierre**
  **63500 Saint Yvoine (FR)**

(74) Representative: **Cabinet Becker et Associés**
**25, rue Louis le Grand**
**75002 Paris (FR)**

(54) **GENES SIGNATURES FOR THE PREDICTION OF PREDISPOSITION TO OBESITY**

(57) The present invention relates to gene expression signatures, as well as methods and kits using said signatures, for predicting or evaluating the predisposition to obesity in a subject. The signature comprises at least GLUL, SFRP4, ITIH5, GPC1, VGLL3 and C1QTNF1 genes. The methods and kits can be used for selecting a subject susceptible to benefit from prevention against obesity.

**EP 4 686 763 A1**

**Description**

**FIELD OF THE INVENTION**

The present invention relates to the field of medicine, in particular of obesity. More specifically, it relates to the determination of a predisposition to obesity.

**BACKGROUND OF THE INVENTION**

Obesity and metabolic diseases have emerged in developed countries as significant health concerns. Obesity is a risk factor for several diseases such as insulin resistance and type II diabetes, cardiovascular diseases such as heart disease and stroke, and some cancers.

According to WHO, in 2022, 2.5 billion adults aged 18 years and older were overweight, including over 890 million adults who were living with obesity. About 16% of adults aged 18 years and older worldwide were obese in 2022. The worldwide prevalence of obesity more than doubled between 1990 and 2022. Susceptibility to metabolic diseases and obesity vary between individuals, with intrinsic and environmental factors contributing to this variation. Epigenetic and genetic factors are both recognized as intrinsic factors playing a role in the development and outcome of obesity and metabolic diseases. However, genetic factors alone are not sufficient to explain predispositions towards obesity and/or metabolic diseases. Epigenetic modifications of the genome lead to changes in the expression of certain genes and thus consecutive expression modifications could be linked to predisposition to metabolic diseases and the development of obesity.

The perinatal period represents a critical window for epigenetic modifications of gene expression. Indeed, a maternal nutritional mismatch during pregnancy and/or in the early weeks/months after birth significantly contributes to the development of obesity, metabolic syndrome, and diabetes. The fetus responds to maternal nutritional stresses through specific adaptations at the cellular and molecular levels, which permanently affect its physiology and metabolism, persisting even when the initial stress has been resolved.

Prior research on factors that characterize predisposition to obesity focused in part on genetic factors, such as genes and SNP markers (WO2011/058232), detection of an alteration in a given gene (WO2005/021787) or of alleles (WO2003/097683) that are associated with obesity.

Obesity is largely preventable and manageable. People may be able to reduce their risk by adopting preventive interventions such as health diet and lifestyles.

Therefore, there is an urgent need to detect epigenetic-related individual predisposition to obesity and metabolic diseases, before the onset of the disease or its symptoms, in order to provide or improve prevention and treatment for these conditions.

**SUMMARY OF THE INVENTION**

To answer this need, the inventors have identified genes that can be used to predict the predisposition to obesity in a subject, before the onset of the disease or its symptoms.

The invention related to the use of expression level of a gene signature comprising or consisting of GLUL, SFRP4, ITIH5, GPC1, VGLL3 and C1QTNF1 as a biomarker in a biological sample for determining a predisposition to develop obesity in a subject.

The invention also relates to a method for predicting if a subject has a predisposition to develop obesity, said method comprising providing a biological sample from said subject and determining an expression level of the genes GLUL, SFRP4, ITIH5, GPC1, VGLL3 and C1QTNF1, thereby predicting whether said subject has a predisposition to develop obesity.

Particularly, in the use or method of the invention, the under-expression of GLUL, and/or the over-expression of genes SFRP4, ITIH5, GPC1, VGLL3 and C1QTNF1 are indicative of a predisposition to develop obesity in said subject.

In one aspect, the gene signature further comprises 1, 2, 3, 4, 5, 6, 7, 8, 9, 10, 15, 20, 25, 30, 35, 40, 45, 50, 55, 60 or 65 additional genes selected from the group consisting of PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, ANXA2, SMPDL3A, RBBP6, EYA2, ARL4A, PALMD, EPS15, TPCN1, PRELP, FXN, AP1S1, PLCE1, LEO1, PCBP1, SNTA1, MDFIC, ANKRD39, NFIL3, METTL16, PPP1CC, SERPINF1, DIS3, STIM2, EMG1, CPT1A, and EHHADH.

In another aspect, the method further comprises determining the expression level of 1, 2, 3, 4, 5, 6, 7, 8, 9, 10, 15, 20, 25, 30, 35, 40, 45, 50, 55, 60 or 65 additional genes selected from the group consisting of PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, ANXA2, SMPDL3A, RBBP6, EYA2, ARL4A, PALMD, EPS15, TPCN1, PRELP, FXN, AP1S1, PLCE1, LEO1, PCBP1, SNTA1, MDFIC, ANKRD39, NFIL3, METTL16, PPP1CC, SERPINF1, DIS3, STIM2, EMG1, CPT1A, and EHHADH.

The additional genes of the uses or methods of the invention may belong one of the following sets:

a) the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, and AATF;

b) the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, and NCBP1;

c) the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, and PAMR1;

d) the genes GLUL, SFRP4, ITIH5, GPC1, VGLL3, C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, and CERCAM;

e) the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, and SULF2;

f) the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, and LRRC20;

g) the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, and ANXA2;

h) the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, ANXA2, SMPDL3A, RBBP6, EYA2, ARL4A, PALMD, EPS15, TPCN1, PRELP, FXN, AP1S1, PLCE1, LEO1, PCBP1, SNTA1, MDFIC, ANKRD39, and NFIL3; or

i) the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, ANXA2, SMPDL3A, RBBP6, EYA2, ARL4A, PALMD, EPS15, TPCN1, PRELP, FXN, AP1S1, PLCE1, LEO1, PCBP1, SNTA1, MDFIC, ANKRD39, NFIL3, METTL16, PPP1CC, SERPINF1, DIS3, STIM2, EMG1, CPT1A, and EHHADH.

In a further aspect,

- In the set a), underexpression of PHB2, SFXN2, IRS1, AACS, MTMR4, AATF, SLC25A38, DNAJB9, HOXA5, NDUFS2, BAG4, and NCBP1 and overexpression of PCOLCE2, LHFPL2, TUBA1A, S100A11, ADAP2, SYNPO2, HEBP1, COL6A1, FADS3, MAN1B1, and PID1 are indicative of a predisposition to develop obesity in said subject; or
- in the set b), underexpression of PHB2, SFXN2, IRS1, AACS, MTMR4, AATF, SLC25A38, DNAJB9, HOXA5, NDUFS2, BAG4, and NCBP1 and overexpression of PCOLCE2, LHFPL2, TUBA1A, S100A11, ADAP2, SYNPO2, HEBP1, COL6A1, FADS3, MAN1B1, PID1, COL6A2, PMEPA1 , and HTRA3 are indicative of a predisposition to develop obesity in said subject; or
- in the set c), underexpression of PHB2, SFXN2, IRS1, AACS, MTMR4, AATF, SLC25A38, DNAJB9, HOXA5, NDUFS2, BAG4, NCBP1, DGAT1, and SIK3, and overexpression of PCOLCE2, LHFPL2, TUBA1A, S100A11, ADAP2, SYNPO2, HEBP1, COL6A1, FADS3, MAN1B1, PID1, COL6A2, PMEPA1, HTRA3, RNASEL, and PAMR1 are indicative of a predisposition to develop obesity in said subject; or
- in the set d), underexpression of PHB2, SFXN2, IRS1, AACS, MTMR4, AATF, SLC25A38, DNAJB9, HOXA5, NDUFS2, BAG4, NCBP1, DGAT1, SIK3, PPP1R15B, and NT5DC1 and overexpression of PCOLCE2, LHFPL2, TUBA1A, S100A11, ADAP2, SYNPO2, HEBP1, COL6A1, FADS3, MAN1B1, PID1, COL6A2, PMEPA1, HTRA3, RNASEL, PAMR1, PAM, STAT6, MAN2B1, and CERCAM are indicative of a predisposition to develop obesity in said subject; or
- in the set e), underexpression of PHB2, SFXN2, IRS1, AACS, MTMR4, AATF, SLC25A38, DNAJB9, HOXA5, NDUFS2, BAG4, NCBP1, DGAT1, SIK3, PPP1R15B, NT5DC1, SLC25A34, PPP1R1A, SLC27A1, TST, PDSS1, and LARP1 and overexpression of PCOLCE2, LHFPL2, TUBA1A, S100A11, ADAP2, SYNPO2, HEBP1, COL6A1, FADS3, MAN1B1, PID1, COL6A2, PMEPA1, HTRA3, RNASEL, PAMR1, PAM, STAT6, MAN2B1, CERCAM, ANXA1, COL18A1, ANKRD29, METRN, MAN2B2, IAH1, RTN2, FLOT1, and SULF2 are indicative of a predisposition to develop obesity in said subject; or
- in the set f), underexpression of PHB2, SFXN2, IRS1, AACS, MTMR4, AATF, SLC25A38, DNAJB9, HOXA5, NDUFS2, BAG4, NCBP1, DGAT1, SIK3, PPP1R15B, NT5DC1, SLC25A34, PPP1R1A, SLC27A1, TST, PDSS1, LARP1, CSTF1, GDE1, UBLCP1, NUP54, ATAD3A, LCMT2, and BRWD1 and overexpression of PCOLCE2, LHFPL2, TUBA1A, S100A11, ADAP2, SYNPO2, HEBP1, COL6A1, FADS3, MAN1B1, PID1, COL6A2, PMEPA1, HTRA3, RNASEL, PAMR1, PAM, STAT6, MAN2B1, CERCAM, ANXA1, COL18A1, ANKRD29, METRN, MAN2B2, IAH1, RTN2, FLOT1, SULF2, HIP1, REEPS, and LRRC20 are indicative of a predisposition to develop obesity in said subject; or
- in the set g), underexpression of PHB2, SFXN2, IRS1, AACS, MTMR4, AATF, SLC25A38, DNAJB9, HOXA5, NDUFS2, BAG4, NCBP1, DGAT1, SIK3, PPP1R15B, NT5DC1, SLC25A34, PPP1R1A, SLC27A1, TST, PDSS1, LARP1, CSTF1, GDE1, UBLCP1, NUP54, ATAD3A, LCMT2, BRWD1, LSM1, CSE1L, SLTM, JTB, HSPA9, RARRES1, and INTS4 and overexpression of PCOLCE2, LHFPL2, TUBA1A, S100A11, ADAP2, SYNPO2, HEBP1, COL6A1, FADS3, MAN1B1, PID1, COL6A2, PMEPA1, HTRA3, RNASEL, PAMR1, PAM, STAT6, MAN2B1, CERCAM, ANXA1, COL18A1, ANKRD29, METRN, MAN2B2, IAH1, RTN2, FLOT1, SULF2, HIP1, REEP5, LRRC20, PGM2L1, GPX7, MAP1LC3A, ARHGEF6, RSU1, AHNAK, SPATA6, PPFIA3, LGALS1, and ANXA2 are indicative of a predisposition to develop obesity in said subject; or
- in the set h), underexpression of PHB2, SFXN2, IRS1, AACS, MTMR4, AATF, SLC25A38, DNAJB9, HOXA5, NDUFS2, BAG4, NCBP1, DGAT1, SIK3, PPP1R15B, NT5DC1, SLC25A34, PPP1R1A, SLC27A1, TST, PDSS1, LARP1, CSTF1, GDE1, UBLCP1, NUP54, ATAD3A, LCMT2, BRWD1, LSM1, CSE1L, SLTM, JTB, HSPA9, RARRES1, INTS4, RBBP6, FXN, LEO1, PCBP1, and NFIL3 and overexpression of PCOLCE2, LHFPL2, TUBA1A, S100A11, ADAP2, SYNPO2, HEBP1, COL6A1, FADS3, MAN1B1, PID1, COL6A2, PMEPA1, HTRA3, RNASEL, PAMR1, PAM, STAT6, MAN2B1, CERCAM, ANXA1, COL18A1, ANKRD29, METRN, MAN2B2, IAH1, RTN2, FLOT1, SULF2, HIP1, REEP5, LRRC20, PGM2L1, GPX7, MAP1LC3A, ARHGEF6, RSU1, AHNAK, SPATA6, PPFIA3, LGALS1, ANXA2, SMPDL3A, EYA2, ARL4A, PALMD, EPS15, TPCN1, PRELP, AP1S1, PLCE1, SNTA1, MDFIC, and ANKRD39 are indicative of a predisposition to develop obesity in said subject; or
- in the set i), underexpression of PHB2, SFXN2, IRS1, AACS, MTMR4, AATF, SLC25A38, DNAJB9, HOXA5, NDUFS2, BAG4, NCBP1, DGAT1, SIK3, PPP1R15B, NT5DC1, SLC25A34, PPP1R1A, SLC27A1, TST, PDSS1, LARP1, CSTF1, GDE1, UBLCP1, NUP54, ATAD3A, LCMT2, BRWD1, LSM1, CSE1L, SLTM, JTB, HSPA9, RARRES1, INTS4, RBBP6, FXN, LEO1, PCBP1, NFIL3, METTL16, PPP1CC, DIS3, EMG1, and EHHADH and overexpression of PCOLCE2, LHFPL2, TUBA1A, S100A11, ADAP2, SYNPO2, HEBP1, COL6A1, FADS3, MAN1B1, PID1, COL6A2, PMEPA1, HTRA3, RNASEL, PAMR1, PAM, STAT6, MAN2B1, CERCAM, ANXA1, COL18A1, ANKRD29, METRN, MAN2B2, IAH1, RTN2, FLOT1, SULF2, HIP1, REEP5, LRRC20, PGM2L1, GPX7, MAP1LC3A, ARHGEF6, RSU1, AHNAK, SPATA6, PPFIA3, LGALS1, ANXA2, SMPDL3A, EYA2, ARL4A, PALMD, EPS15, TPCN1, PRELP, AP1S1, PLCE1, SNTA1, MDFIC, ANKRD39, SERPINF1, STIM2, and CPT1A are indicative of a predisposition to develop obesity in said subject.

In an aspect, the biological sample for the uses or methods of the invention comprises adipose tissue, in particular an adipose tissue biopsy sample.

In an aspect, the expression level of genes is detected and/or quantified at the mRNA level. In a further aspect, the mRNA level is detected and/or quantified by transcription-mediated amplification, northern blot, reverse transcription polymerase chain reaction (RT-PCR), Serial Analysis of Gene Expression (SAGE), DNA microarray, tiling array, digital PCR, *in situ* hybridization, or RNA sequencing (RNA-Seq).

Optionally, the expression level of genes is compared to a reference of expression level, in particular the expression level in a population of lean subjects without a predisposition to obesity.

The invention also related to a kit comprising analysis means for detecting and/or quantifying in a biological sample the expression level of the genes GLUL, SFRP4, ITIH5, GPC1, VGLL3 and C1QTNF1. In a preferred aspect, the kit is suitable for detecting and/or quantifying the expression level of less than 150 genes.

In an aspect, said kit further comprises analysis means for detecting and/or quantifying in a sample the expression level of

- the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, and AATF;
- the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, and NCBP1;
- the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, and PAMR1;
- the genes GLUL, SFRP4, ITIH5, GPC1, VGLL3, C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, and CERCAM;
- the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, and SULF2;
- the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, and LRRC20;
- the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, and ANXA2;
- the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, ANXA2, SMPDL3A, RBBP6, EYA2, ARL4A, PALMD, EPS15, TPCN1, PRELP, FXN, AP1S1, PLCE1, LEO1, PCBP1, SNTA1, MDFIC, ANKRD39, and NFIL3; or
- the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, ANXA2, SMPDL3A, RBBP6, EYA2, ARL4A, PALMD, EPS15, TPCN1, PRELP, FXN, AP1S1, PLCE1, LEO1, PCBP1, SNTA1, MDFIC, ANKRD39, NFIL3, METTL16, PPP1CC, SERPINF1, DIS3, STIM2, EMG1, CPT1A, and EHHADH.

In an aspect, the invention relates to the use of the kit as disclosed herein for determining if a subject has a predisposition to develop obesity.

Finally, the present invention relates to a method for monitoring a subject comprising predicting if the subject has a predisposition to develop obesity by the method of the invention, and selecting the subject having said predisposition as susceptible to benefit from a preventive action against obesity.

## DESCRIPTION OF THE FIGURES

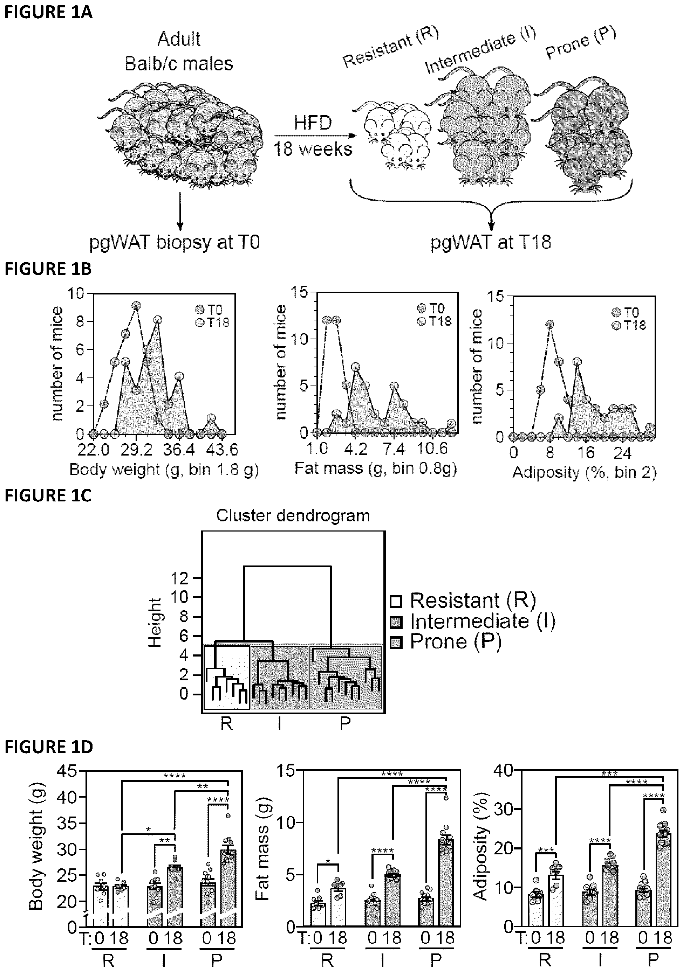

**Figure 1A: experimental design for the innate variability model.** 4-month-old BALB/c males mice were monitored (n=27). At TO, perigonadal WAT (pgWAT) biopsies were taken. Animals were allowed to recover for 1 week. Subsequently, they were fed an experimental high-fat diet (HFD) for 18 weeks after which they were sacrificed (T18) to harvest perigonadal WAT. Schematically, at the end of the HFD challenge, the mice exhibit different sensitivities to obesity and will thus be categorized into 3 groups: R mice are resistant to Diet-induced Obesity (DIO), I mice have an intermediate phenotype, and P mice are prone to DIO.

**Figure 1B: Phenotypic characterization of mice based on sensitivity to DIO.** Individual parameters such as body weight (g), fat mass (g), and adiposity (%) were measured at TO (before HFD-challenge) and at T18 (after 18 weeks consuming a HFD). Graphs show the distribution of the number of mice according to each parameter. Gray dots represent the animal before HFD-challenge (TO), filled dots represent animals after HFD-challenge (T18). In each case, three groups of mice exhibiting different behavior regarding diet-induced changes in physical parameters are clearly identified.

**Figure 1C: Hierarchical Clustering Analysis (HCA) considering all the physical parameters measured.** Body weight, fat mass, and adiposity at T18, delta% body weight, delta% fat mass, and delta% adiposity were used to classify mice in three groups: R mice are resistant to DIO (n=7), P mice are prone to DIO (n=11), and I mice exhibit an intermediate phenotype (n=9).

**Figure ID: Body weight, fat** mass **and adiposity before and after HFD-challenge for each group R, I and P.** Body weight (g), fat mass (g), and adiposity (%) at TO (gray dots) and T18 (pink dots) for R, I, and P groups (n= 7, 9, 11 respectively). Bars represent the mean for each group and error bars correspond to SEM. One-way ANOVA p-value is indicated as follow: * $p \leq 0.05$; ** $p \leq 0.01$; *** $p \leq 0.001$; **** $p \leq 0.0001$.

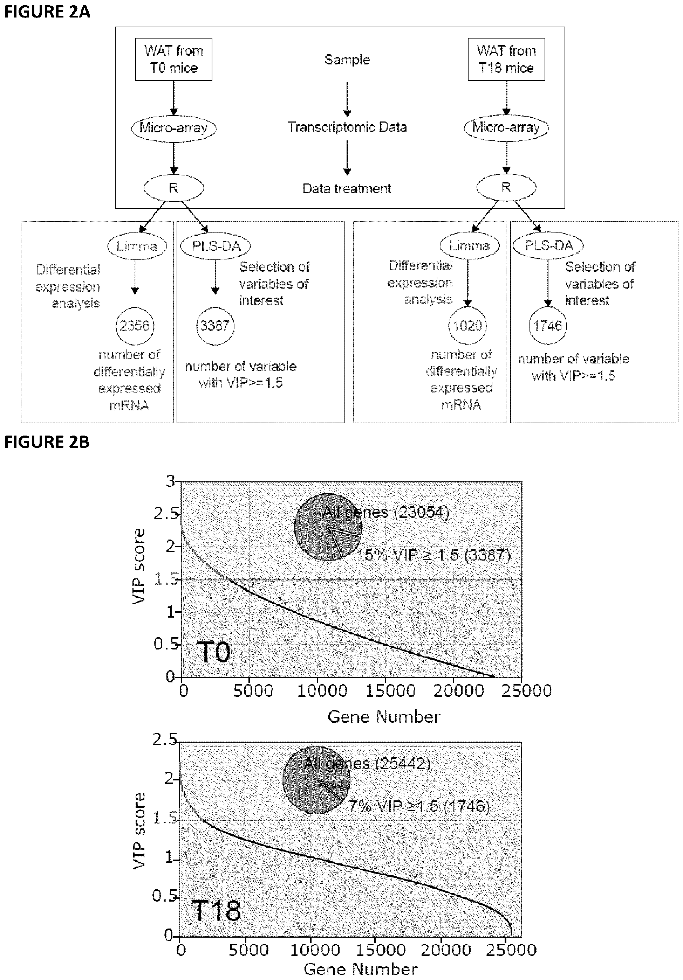

**Figure 2A: Workflow to** select genes **of** interest. Micro-arrays were performed on RNA extracted from pgWAT harvested at TO and T18 from mice in the three groups R (n= 8), I (n= 7), P (n= 12). Raw data were treated with the R package Limma prior to differential expression analysis (green part of the workflow). The mixOmics R package was used for PLS-DA (blue part of the workflow) in order to identify important genes based on their VIP scores. A VIP score is a measure of a variable's importance in the PLS-DA model. It summarizes the contribution of a variable makes to the model.

**Figure 2B: Variable of importance in projection (VIP) scores for each gene used in the PLS-DA.** Genes contributing meaningfully to the PLS-DA model with a VIP score >1.5 constitute respectively 15% and 7% of the genes tested for TO and T18 data (inset pie-chart). The y-axis corresponds to the VIP scores for each variable on the X-axis. Red part of the curve corresponds to genes with the highest VIP scores (>=1.5) and thus are the most contributory genes in class discrimination in the PLS-DA model.

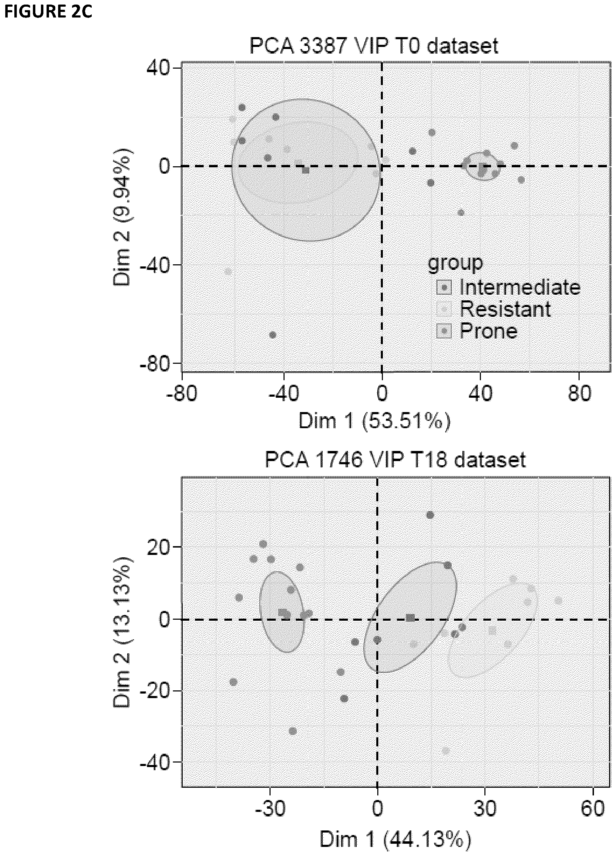

**Figure 2C: Principal Component Analysis (PCA).** PCA was performed using the set of 1746 mRNA selected by PLS-DA at T18 and 3387 mRNA selected by PLS-DA at TO. Data from groups I (green dots), R (yellow dots), and P (pink dots) are plotted, along the first (X) and second (Y) principal components. Ellipses represent the 95% confidence interval and squares represent the barycenter of each group.

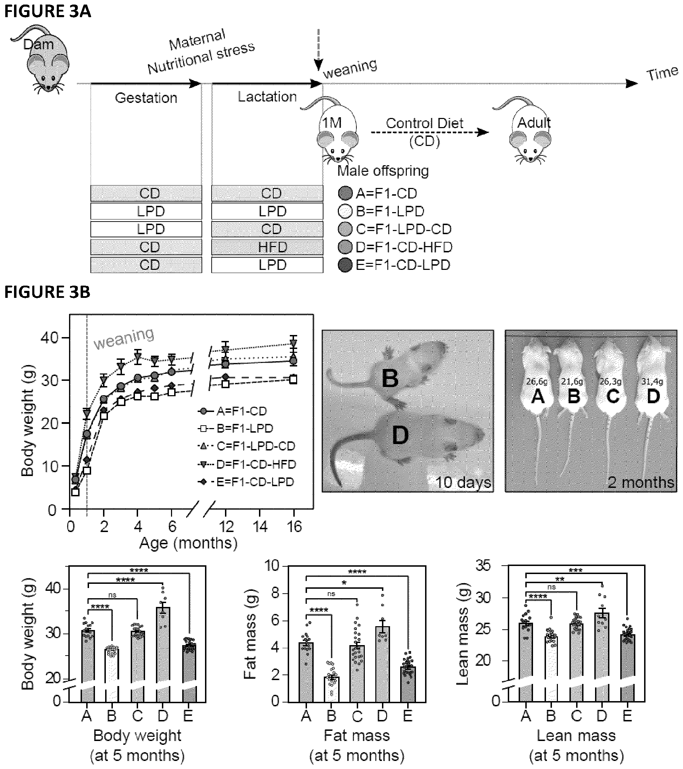

**Figure 3A: Experimental design for the nutritional programming model.** Two-month-old virgin BALB/c female mice fed a A03 chow diet were mated with BALB/c males. Gestating animals were isolated when a vaginal plug was detected, and fed the experimental diet as indicated. LPD and CD are isocaloric. At parturition, dams were fed with the experimental diets indicated. Litters of different sizes were obtained from each group of pregnant female. Since the litter size is important in the offspring life trajectory, to avoid extreme litter size, only litters that have a total number of pups comprised between 4 and 10 were considered. After weaning, male offspring from each group were housed individually with free access to CD. Moreover, to obviate any litter effects, animals used for each experiment were randomly chosen from different litters, and only a limited number of animals (n = 1 to 2) was used from each litter.

**Figure 3B: Phenotypic characterization of male offspring born from dams fed various diets during gestation and lactation.** (upper left panel) Male offspring born from dams fed experimental diets during gestation and lactation were weighed at Post-Natal Day 10 (PND10), every month from 1 to 6 months, and at 12 and 16 months. The results presented are the average of 3 independent experiments. (upper right panel) Representative mice from groups B and D at PND10 and from groups A, B, C, and D at 2 months. Body weight is indicated for each mouse. (bottom panel) Body composition parameters of 5-month-old A, B, C, D, and E male mice. Body weight, fat mass and lean mass are indicated in grams. All values correspond to mean +/- SEM for at least n=8 /group. One-way ANOVA p-value is indicated as follow: * $p \leq 0.05$; ** $p \leq 0.01$; *** $p \leq 0.001$; **** $p \leq 0.0001$.

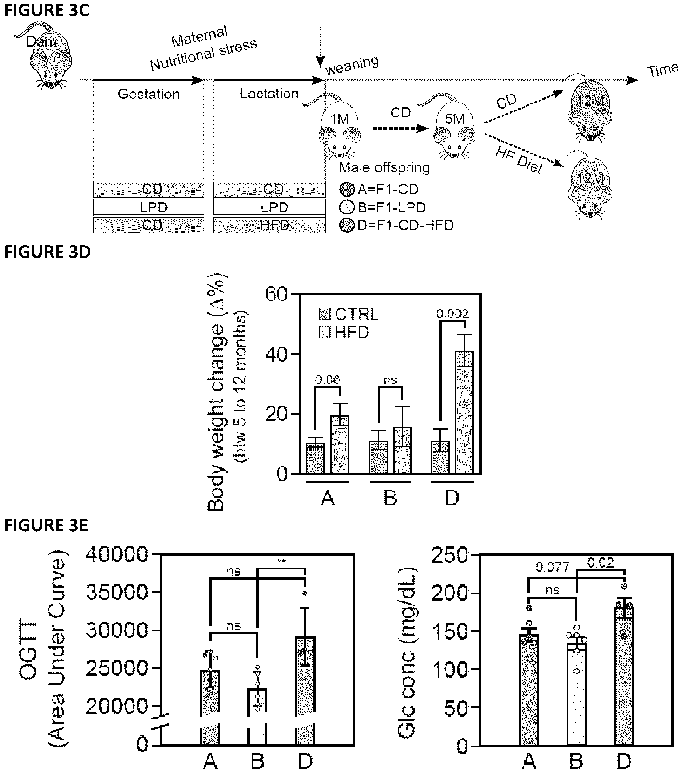

**Figure 3C: Experimental design for the HFD-challenge on groups A, B, and D.** 5-month-old male offspring from groups A, B, and D were fed either a CD or a HFD from the age of 5 to 12 months (7-month HFD-challenge).

**Figure 3D: Body weight gain during HFD-challenge.** Body weight gain between the beginning (5 months) and the end (12 months) of the HFD-challenge was calculated and expressed as the weight gain relative to the initial weight, given in %. n>=4/group, One-way ANOVA p-value is indicated.

**Figure 3E: OGTT measured after HFD-challenge.** 12-month-old male mice from groups A, B, or D fed a HFD for 5 months were subjected to an Oral Glucose tolerance test. Area Under curve and starved glucose concentration were measured. n>=4, One-way ANOVA p-value is indicated is indicated as follow: ns=not significant, ** p<0.01.

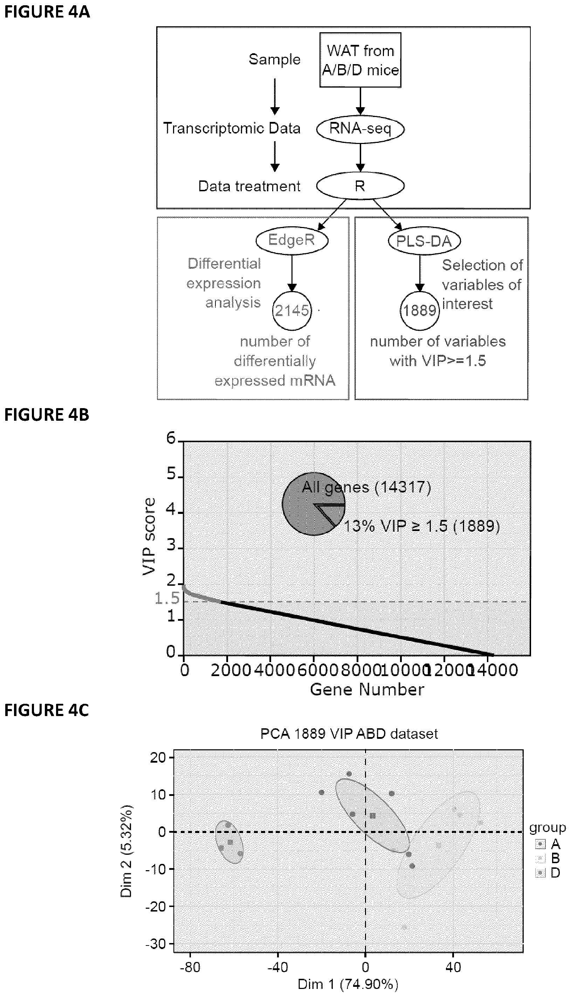

**Figure 4A: Workflow to select genes of interest.** RNA sequencing was performed on RNA extracted from pg-WAT harvested from 5-month-old mice from the three groups A (n= 6), B (n= 5), and D (n= 3). Raw data were treated with the R package EdgeR prior to differential expression analysis (green part of the workflow). The mixOmics R package was used for PLS-DA (blue part of the workflow) to identify important genes based on their VIP scores.

**Figure 4B: Variable of importance in projection (VIP) scores for each gene used in the PLS-DA.** Genes contributing meaningfully to the PLS-DA model with a VIP score >1.5 constitute 13% of the genes tested (inset pie-chart). The y-axis indicates the VIP scores for each variable indicated on the X-axis. Red dots indicate variables with the highest VIP scores ($\geq 1.5$) and thus contributing the most to class discrimination in the PLS-DA model.

**Figure 4C: Principal Component Analysis (PCA).** PCA was performed using the set of 1889 mRNA selected by PLS-DA. Data from groups A (green dots), B (yellow dots), and D (pink dots) are plotted along the first (X) and second (Y) principal components. Ellipses represent the 95% confidence interval and squares represent the barycenter of each group.

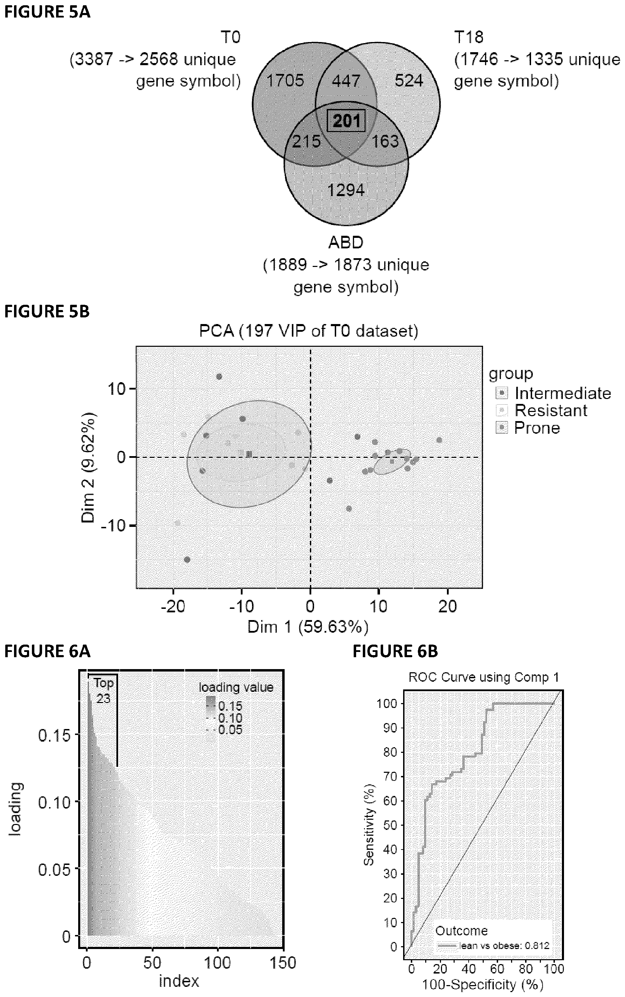

**Figure 5A: Strategy of identification of candidate genes for the prediction of a predisposition to obesity.** To identify the most robust predictive genes, the three gene lists (containing 1889, 1746, and 3387 genes from the three datasets ABD, TO and T18) obtained by PLS-DA were compared and a list of 201 genes was identified. Among these 201 common genes, 4 presented an adjusted q-value >= 0.05 for all three data sets (ABD, TO, T18) and were therefore eliminated, leaving 197 selected genes.

**Figure 5B: Principal Component Analysis (PCA).** PCA was performed using the expression of the 197 selected genes from the first model at TO. Data from groups I (green dots), R (yellow dots), and P (pink dots) are plotted with respect to the first (X) and second (Y) principal components. Ellipses represent the 95% confidence interval and squares represent the barycenter of each group.

**Figure 6A: Distribution of genes according to their loading values on the seven gene expression datasets.** Three mouse data sets were used: nutritional programing model (groups A and D), innate variability model at TO (group I and P) and innate variability model at T18 (group I and P) together with four human datasets from publicly available resources (GEOD-2508: microarray data obtained from isolated adipocytes of subcutaneous adipose deposits from Pima Indians, male or female and lean or obese (Lillioja, S., Bogardus, C., 1988. Diabetes/Metabolism Reviews 4(5): 517-40, Doi: 10.1002/dmr.5610040508). Cohort MTAB-6728 contains a large number of lean and obese individuals without specifications on biological sex (Bjune, J.-I., et al., 2019. International Journal of Obesity 43(11): 2151-62, Doi: 10.1038/s41366-018-0275-y). Cohort GSE-166047 consists of lean and obese females (Rey, F., et al., 2021. International Journal of Molecular Sciences 22(4): 1989, Doi: 10.3390/ijms22041989). E-GEOD-9624 is a cohort of children for whom omental adipose tissue samples were analyzed (Aguilera, C., et al., 2015. International Journal of Molecular Sciences 16(12): 7723-37, Doi: 10.3390/ijms16047723).

**Figure 6B: ROC curve and AUC from the MINT PLS-DA for the seven gene expression datasets.** In a ROC curve the true positive rate (i.e. the proportion of correctly predicted positive instances relative to all actual positive instances = Sensitivity) is plotted in function of the false positive rate (i.e. the proportion of incorrectly predicted positive instances relative to all actual negative instances = 100-Specificity). The numerical output indicated is the area under curve (AUC) measuring the overall performance of the model. It quantifies the ability of the model to discriminate between positive and negative classes. A higher AUC indicates better performance. AUC values greater than 0.8 are often considered very good and suggest a model with strong discriminative power.

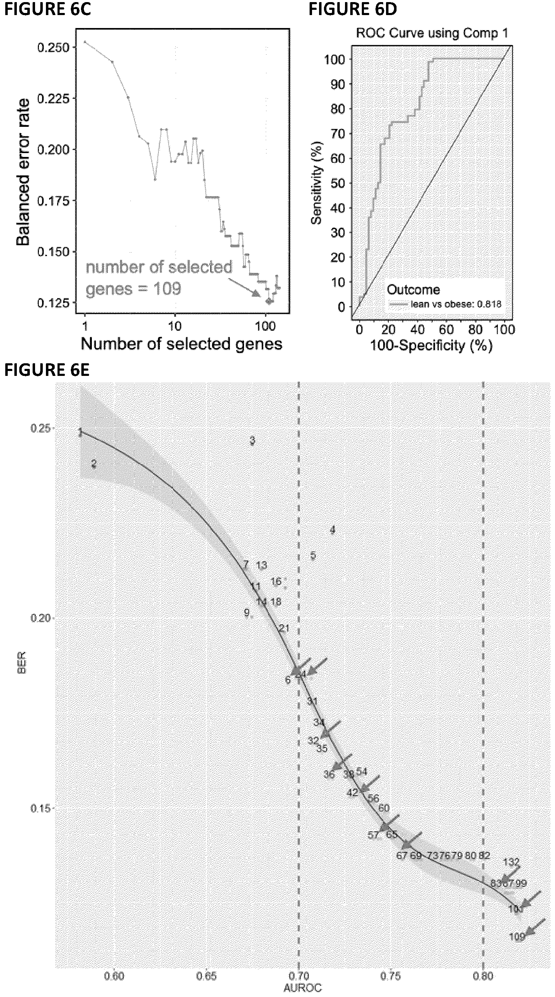

**Figure 6C: Tuning keepX in MINT.splsda performed on the seven gene expression datasets.** The line represents the balanced error rate (y-axis) for component 1 across all tested keepX values (x-axis). Is called keepX the minimum number of gene necessary to keep a model with at least the same performance than the initial model. The diamond indicates the optimal keepX value which achieves the lowest classification error rate as determined with a one-sided t-test across the studies.

**Figure 6D: ROC curve and AUC from the MINT.splsda performed on the seven gene expression datasets**. **Figure 6E: BER (y-axis) depending on the AUROC (x-axis) for each number of genes kept in the model, and fitted curve.** Red arrows indicate thresholds for the number of genes involved in the model at which the BER substantially shifts.

## DETAILED DESCRIPTION OF THE INVENTION

The inventors identified gene signatures that allow for the prediction of a predisposition to obesity. Their approach to identify genes that indicate a predisposition to obesity relied on several experiments. First, interindividual variability in obesity predisposition was examined in a murine model. Genetically homogeneous mice were fed a high-fat diet (HFD) for 18 weeks. At the end of the experiment, three groups of mice could be defined: certain mice exhibited minimal weight gain and were thus classified as DIO-resistant, whereas others exhibited excessive weight gain and were considered prone to DIO. An intermediate group with intermediate weight gain was also identified. Gene expression (measured *via* RNA-Seq) in white adipose tissue from these mice before the start (T0) and at the end (T18) of the HFD challenge were compared for the resistant, prone and intermediate groups. Following this analysis, 3387 mRNA species at TO and 1746 mRNA species at T18 were selected as potential variables of interest.

Secondly, the inventors assessed the effects of the type and timing of maternal nutritional stress on predisposition to diet-induced obesity among offspring. In the murine model, mothers were fed different diets (Low-Protein Diet, Control Diet or Hit-Fat Diet) during gestation and/or lactation. At weaning, their offspring were then fed a control diet, and their biological parameters (body weight, fat mass, and adiposity) were measured for several months. Results showed differences in body weight linked to maternal nutritional status. Here, gene expression was analyzed in white adipose tissue from the offspring. 1889 mRNA species were identified as capable of distinguishing individuals between three groups of offspring mice whose mothers had received different diets during gestation and/or lactation.

Finally, 201 discriminant genes common to the two models were identified, and it was assessed if the human orthologs of the candidate genes identified could also be considered as good candidates to be predictive of susceptibility to obesity in humans. Four independent external datasets derived from previous studies on humans were used, with each human dataset used being composed of two groups (lean and obese) for which transcriptomic analysis was performed on white adipose tissue. Statistical analysis revealed sets of genes whose expression can be used as a signature for the prediction of obesity. A minimal signature of 6 genes was identified as a biomarker of a predisposition to obesity. This minimal signature can be enriched in several signatures, for instance of 23, 32, 36, 42, 57, 67, 84 and 109 genes.

The identified gene signatures disclosed herein allow for the evaluation of a predisposition to obesity.

### Definitions

For better understanding of the present invention, certain terms are defined thereafter. Additional definitions are presented throughout the detailed description.

Obesity shall be construed as any condition of abnormal or excessive fat accumulation in adipose tissue, to the extent that health may be impaired. According to the WHO definition and for adults, overweight is defined as a subject having a BMI (body mass index) greater than or equal to 25 and obesity as a subject having a BMI greater than or equal to 30. For children under 5 years of age, overweight is defined as a subject having weight-for-height greater than 2 standard deviations above WHO Child Growth Standards median and obesity as a subject having weight-for-height greater than 3 standard deviations. For children aged between 5-19 years, overweight is defined as a subject having a BMI-for-age greater than 1 standard deviation above the WHO Growth Reference median and obesity as a subject having a BMI-for-age greater than 2 standard deviations.

The term "gene" as used herein refers to a nucleic acid sequence that comprises coding sequences necessary for producing a polypeptide or precursor. Control sequences that direct and/or control expression of the coding sequences may also be encompassed by the term "gene" in some instances. The polypeptide or precursor may be encoded by a full length coding sequence or by a portion of the coding sequence. A gene may contain one or more modifications in either the coding or the untranslated regions that could affect the biological activity or the chemical structure of the polypeptide or precursor, the rate of expression, or the manner of expression control. The gene may constitute an uninterrupted coding sequence or it may include one or more subsequences. The term "gene" as used herein includes the genes identified in Table A. The sequences of the genes listed herein can readily be obtained by one of skill in the art from publicly available databases, such as but not limited to the GenBank database maintained by the National Center for Biotechnology (NCBI), for example, by searching using the provided gene symbols. These gene symbols are recognized by databases including but not limited to HGNC, Entrez Gene, UniProtKB/Swiss-Prot, OMIM, GeneLoc, and/or Ensembl; all aliases listed herein are defined by the GeneCards database.

The term "nucleic acid" as used herein, refers to a molecule comprised of one or more nucleotides, for example, ribonucleotides, deoxyribonucleotides, or both. The term includes monomers and polymers of nucleotides, with the nucleotides being bound together, in the case of the polymers, in sequence, typically via 5' to 3' linkages, although alternative linkages are also contemplated in some embodiments. The nucleotide polymers may be single or double-stranded. The nucleotides may be naturally occurring or may be synthetically produced analogs that are capable of forming base-pair relationships with naturally occurring base pairs. Examples of non-naturally occurring bases that are capable of forming base-pairing relationships include, but are not limited to, aza and deaza pyrimidine analogs, aza and deaza purine analogs, and other heterocyclic base analogs, wherein one or more of the carbon and nitrogen atoms of the pyrimidine rings have been substituted by heteroatoms, e.g., oxygen, sulphur, selenium, phosphorus, and the like.

As used herein, the term "gene expression" refers to the process of converting genetic information encoded in a gene into RNA (e.g., mRNA, rRNA, tRNA, or snRNA) through "transcription" of the gene (e.g., via the enzymatic action of an RNA polymerase), and for protein encoding genes, into protein through "translation" of mRNA.

Overexpression of a gene has its meaning as known in the art here. It can be defined as an increased amount of mRNA or protein encoded by the given gene when compared to a reference. Likewise, underexpression of a gene has its meaning as known in the art here. It can be defined as an decreased amount of mRNA or protein encoded by the given gene when compared to a reference.

As used herein, a "gene signature" refers to a pattern of gene expression of a selected set of genes that provides a unique identifier of a biological sample. For purposes of this application, a "gene signature" may be a pre-determined combination of nucleic acid or polypeptide sequences (if the genes are protein-coding genes).

As used herein, the terms "marker" and "biomarker", in particular the expression level of certain markers such as proteins or peptides markers or mRNA markers encoded by genes, are interchangeable and refer to biological parameters that permit the selection of subjects who will have a predisposition to obesity.

The term "biological sample" means any biological sample derived from an individual, preferably a sample which contains nucleic acids or proteins. Examples of such samples include fluids, tissues, cell samples, organs, biopsies, etc.

As used herein, the term "subject" or "individual" refer to an animal, preferably to a mammal, even more preferably to a human.

## Gene expression signature for determining predisposition to develop obesity

The present invention discloses a gene expression signature useful for determining whether an individual or subject is susceptible to develop obesity, before the onset of symptoms thereof. In a preferred aspect, the expression signature is used for prediction of the susceptibility of an individual or subject to obesity.

The present invention also concerns a method for determining if a subject or individual has a predisposition to develop obesity. The present invention also concerns a method for providing data useful for determining if a subject has a predisposition to develop obesity.

By "predicting" or "prediction" is intended herein the likelihood that an individual will develop or not obesity, before he/she shows symptoms thereof. Predictive methods of the invention can be used clinically to make treatment and/or prevention decisions, and/or to select a subject susceptible to benefit from a preventive action against obesity.

Preferably, the subject is a human being. Optionally, the subject can be an adult. Optionally, the subject can be a child.

The subject is not obese. Optionally, the subject can be overweight. Optionally, the subject is not overweight. Optionally, the subject has no family history of obesity. Optionally, the subject has a family history of obesity.

The main objective of the present invention is to develop a prognosis or prediction method based on the detection and/or quantification of certain markers, in particular the expression level of certain markers such as proteins or peptides markers or mRNA markers encoded by genes. The methods of the invention comprise assaying a set of markers in a biological sample from the subject, wherein the detection of the level of expression of the markers, especially under-expression or overexpression of some or all markers, is indicative of a predisposition to develop obesity.

Optionally, the biological sample is a biopsy. Preferably, the biological sample is an adipose tissue biopsy, for example, a subcutaneous adipose tissue biopsy or a visceral adipose tissue biopsy. Alternatively, the biological sample can be a biopsy of another tissue from the subject or a biological fluid. For instance, the biological sample could be skin or blood.

Optionally, the markers are detected and quantified at the protein level, and are proteins or peptides encoded by the genes of the gene signature. Preferably, the markers are detected and quantified at the mRNA level, and are mRNA or fragments thereof encoded by the genes of the gene signature.

The set of markers to be assayed, in particular detected and/or quantified, in a biological sample, for example an adipose tissue biopsy sample, may comprise or consist in 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100, 105 or all markers selected in the group consisting of the proteins or mRNA encoded by the following genes: AACS, AATF, ADAP2, AHNAK, ANKRD29, ANKRD39, ANXA1, ANXA2, AP1S1, ARHGEF6, ARL4A, ATAD3A, BAG4, BRWD1, C1QTNF1, CERCAM, COL18A1, COL6A1, COL6A2, CPT1A, CSE1L, CSTF1, DGAT1, DIS3, DNAJB9, EHHADH, EMG1, EPS15, EYA2, FADS3, FLOT1, FXN, GDE1, GLUL, GPC1, GPX7, HEBP1, HIP1, HOXA5, HSPA9, HTRA3, IAH1, INTS4, IRS1, ITIH5, JTB, LARP1, LCMT2, LEO1, LGALS1, LHFPL2, LRRC20, LSM1, MAN1B1, MAN2B1, MAN2B2, MAP1LC3A, MDFIC, METRN, METTL16, MTMR4, NCBP1, NDUFS2, NFIL3, NT5DC1, NUP54, PALMD, PAM, PAMR1, PCBP1, PCOLCE2, PDSS1, PGM2L1, PHB2, PID1, PLCE1, PMEPA1, PPFIA3, PPP1CC, PPP1R15B, PPP1R1A, PRELP, RARRES1, RBBP6, REEP5, RNASEL, RSU1, RTN2, S100A11, SERPINF1, SFRP4, SFXN2, SIK3, SLC25A34, SLC25A38, SLC27A1, SLTM, SMPDL3A, SNTA1, SPATA6, STAT6, STIM2, SULF2, SYNPO2, TPCN1, TST, TUBA1A, UBLCP1 and VGLL3.

In an aspect, the method for determining the predisposition to develop obesity comprises determining the expression level of at least 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100, 105 or all genes of the following list in a biological of a subject: AACS, AATF, ADAP2, AHNAK, ANKRD29, ANKRD39, ANXA1, ANXA2, AP1S1, ARHGEF6, ARL4A, ATAD3A, BAG4, BRWD1, C1QTNF1, CERCAM, COL18A1, COL6A1, COL6A2, CPT1A, CSE1L, CSTF1, DGAT1, DIS3, DNAJB9, EHHADH, EMG1, EPS15, EYA2, FADS3, FLOT1, FXN, GDE1, GLUL, GPC1, GPX7, HEBP1, HIP1, HOXA5, HSPA9, HTRA3, IAH1, INTS4, IRS1, ITIH5, JTB, LARP1, LCMT2, LEO1, LGALS1, LHFPL2, LRRC20, LSM1, MAN1B1, MAN2B1, MAN2B2, MAP1LC3A, MDFIC, METRN, METTL16, MTMR4, NCBP1, NDUFS2, NFIL3, NT5DC1, NUP54, PALMD, PAM, PAMR1, PCBP1, PCOLCE2, PDSS1, PGM2L1, PHB2, PID1, PLCE1, PMEPA1, PPFIA3, PPP1CC, PPP1R15B, PPP1R1A, PRELP, RARRES1, RBBP6, REEP5, RNASEL, RSU1, RTN2, S100A11, SERPINF1, SFRP4, SFXN2, SIK3, SLC25A34, SLC25A38, SLC27A1, SLTM, SMPDL3A, SNTA1, SPATA6, STAT6, STIM2, SULF2, SYNPO2, TPCN1, TST, TUBA1A, UBLCP1 and VGLL3.

More specifically, the method is further characterized in that

- the overexpression of at least 1,2,3,4, 5, 6, 7, 8, 9, 10, 11, 12, 13, 14, 15, 16, 17, 18, 19, 20, 25, 30, 35, 40, 45, 50, 55, 60, or all genes of the following list: SFRP4, ITIH5, GPC1, VGLL3, C1QTNF1, PCOLCE2, LHFPL2, TUBA1A, S100A11, ADAP2, SYNPO2, HEBP1, COL6A1, FADS3, MAN1B1, PID1, COL6A2, PMEPA1, HTRA3, RNASEL, PAMR1, PAM, STAT6, MAN2B1, CERCAM, ANXA1, COL18A1, ANKRD29, METRN, MAN2B2, IAH1, RTN2, FLOT1, SULF2, HIP1, REEP5, LRRC20, PGM2L1, GPX7, MAP1LC3A, ARHGEF6, RSU1, AHNAK, SPATA6, PPFIA3, LGALS1, ANXA2, SMPDL3A, EYA2, ARL4A, PALMD, EPS15, TPCN1, PRELP, AP1S1, PLCE1, SNTA1, MDFIC, ANKRD39, SERPINF1, STIM2, and CPT1A;
and/or
- the underexpression of at least 1,2,3,4, 5, 6, 7, 8, 9, 10, 11, 12, 13, 14, 15, 16, 17, 18, 19, 20, 25, 30, 35, 40, 45, or all genes of the following list: GLUL, PHB2, SFXN2, IRS1, AACS, MTMR4, AATF, SLC25A38, DNAJB9, HOXA5, NDUFS2, BAG4, NCBP1, DGAT1, SIK3, PPP1R15B, NT5DC1, SLC25A34, PPP1R1A, SLC27A1, TST, PDSS1, LARP1, CSTF1, GDE1, UBLCP1, NUP54, ATAD3A, LCMT2, BRWD1, LSM1, CSE1L, SLTM, JTB, HSPA9, RARRES1, INTS4, RBBP6, FXN, LEO1, PCBP1, NFIL3, METTL16, PPP1CC, DIS3, EMG1, and EHHADH;

are indicative of a predisposition to develop obesity in a subject.

In a particular aspect, the gene expression signature useful for determining a predisposition to develop obesity in a subject comprises the genes GLUL, SFRP4, ITIH5, GPC1, VGLL3 and C1QTNF1. Accordingly, the invention provides a gene signature comprising or consisting of GLUL, SFRP4, ITIH5, GPC1, VGLL3 and C1QTNF1 as a biomarker in a biological sample for determining a predisposition to develop obesity in a subject, and the use of expression level of said signature for determining a predisposition to develop obesity in a subject. More particularly, in this gene signature, the under-expression of GLUL, and the over-expression of genes SFRP4, ITIH5, GPC1, VGLL3 and C1QTNF1 are indicative of a predisposition to develop obesity in said subject.

In addition to these 6 genes, the gene signature may comprise 1, 2, 3, 4, 5, 6, 7, 8, 9, 10, 15, 20, 25, 30, 35, 40, 45, 50, 55, 60, 65 or more additional genes. Preferably, the additional genes are selected from the group consisting of PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, ANXA2, SMPDL3A, RBBP6, EYA2, ARL4A, PALMD, EPS15, TPCN1, PRELP, FXN, AP1S1, PLCE1, LEO1, PCBP1, SNTA1, MDFIC, ANKRD39, NFIL3, METTL16, PPP1CC, SERPINF1, DIS3, STIM2, EMG1, CPT1A, and EHHADH. Accordingly, in an aspect, the method further comprises determining the expression level of 1, 2, 3, 4, 5, 6, 7, 8, 9, 10, 15, 20, 25, 30, 35, 40, 45, 50, 55, 60 or 65 additional genes selected from the group consisting of PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, ANXA2, SMPDL3A, RBBP6, EYA2, ARL4A, PALMD, EPS15, TPCN1, PRELP, FXN, AP1S1, PLCE1, LEO1, PCBP1, SNTA1, MDFIC, ANKRD39, NFIL3, METTL16, PPP1CC, SERPINF1, DIS3, STIM2, EMG1, CPT1A, and EHHADH.

Optionally, the gene signature further comprise one of the following sets of additional genes:

a) the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, and AATF;

b) the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, and NCBP1;

c) the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, and PAMR1;

d) the genes GLUL, SFRP4, ITIH5, GPC1, VGLL3, C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, and CERCAM;

e) the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, and SULF2;

f) the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, and LRRC20;

g) the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, and ANXA2;

h) the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, ANXA2, SMPDL3A, RBBP6, EYA2, ARL4A, PALMD, EPS15, TPCN1, PRELP, FXN, AP1S1, PLCE1, LEO1, PCBP1, SNTA1, MDFIC, ANKRD39, and NFIL3; and

i) the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, ANXA2, SMPDL3A, RBBP6, EYA2, ARL4A, PALMD, EPS15, TPCN1, PRELP, FXN, AP1S1, PLCE1, LEO1, PCBP1, SNTA1, MDFIC, ANKRD39, NFIL3, METTL16, PPP1CC, SERPINF1, DIS3, STIM2, EMG1, CPT1A, and EHHADH.

Accordingly, the gene signature useful for prediction of obesity in a subject can consist or comprise one of the following sets of genes:

a) the genes GLUL, SFRP4, ITIH5, GPC1, VGLL3, C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, and AATF;

b) the genes GLUL, SFRP4, ITIH5, GPC1, VGLL3, C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, and NCBP1;

c) the genes GLUL, SFRP4, ITIH5, GPC1, VGLL3, C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, and PAMR1;

d) the genes GLUL, SFRP4, ITIH5, GPC1, VGLL3, C1QTNF1, GLUL, SFRP4, ITIH5, GPC1, VGLL3, C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, and CERCAM;

e) the genes GLUL, SFRP4, ITIH5, GPC1, VGLL3, C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, and SULF2;

f) the genes GLUL, SFRP4, ITIH5, GPC1, VGLL3, C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, and LRRC20;

g) the genes GLUL, SFRP4, ITIH5, GPC1, VGLL3, C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, and ANXA2;

h) the genes GLUL, SFRP4, ITIH5, GPC1, VGLL3, C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, ANXA2, SMPDL3A, RBBP6, EYA2, ARL4A, PALMD, EPS15, TPCN1, PRELP, FXN, AP1S1, PLCE1, LEO1, PCBP1, SNTA1, MDFIC, ANKRD39, and NFIL3; and

i) the genes GLUL, SFRP4, ITIH5, GPC1, VGLL3, C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, ANXA2, SMPDL3A, RBBP6, EYA2, ARL4A, PALMD, EPS15, TPCN1, PRELP, FXN, AP1S1, PLCE1, LEO1, PCBP1, SNTA1, MDFIC, ANKRD39, NFIL3, METTL16, PPP1CC, SERPINF1, DIS3, STIM2, EMG1, CPT1A, and EHHADH.

Optionally, the gene signature may further comprise other genes. Preferably, the gene signature does not comprise more than 120, 130, 140, 150, 200, 250 or 300 genes.

The method comprises detecting and quantifying the level of expression of the genes belonging to the gene signature.

More particularly, among the genes of the gene signature, some genes are overexpressed in the subjects having a predisposition to develop obesity and other genes are underexpressed, especially in comparison to a reference of expression level.

In an aspect, the method or the use of a gene signature for determining a predisposition to develop obesity in a subject comprises determining an expression level of GLUL, SFRP4, ITIH5, GPC1, VGLL3 , C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, and AATF. In a preferred aspect thereof, underexpression of PHB2, SFXN2, IRS1, AACS, MTMR4, AATF, SLC25A38, DNAJB9, HOXA5, NDUFS2, BAG4, and NCBP1 and overexpression of PCOLCE2, LHFPL2, TUBA1A, S100A11, ADAP2, SYNPO2, HEBP1, COL6A1, FADS3, MAN1B1, and PID1 are indicative of a predisposition to develop obesity in said subject.

In an aspect, the method or the use of a gene signature for determining a predisposition to develop obesity comprises determining an expression level of GLUL, SFRP4, ITIH5, GPC1, VGLL3 , C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, and NCBP1. In a preferred aspect thereof, underexpression of PHB2, SFXN2, IRS1, AACS, MTMR4, AATF and overexpression of PCOLCE2, LHFPL2, TUBA1A, S100A11, ADAP2, SYNPO2, HEBP1, COL6A1, FADS3, MAN1B1, PID1, COL6A2, PMEPA1 , and HTRA3 are indicative of a predisposition to develop obesity in said subject.

In an aspect, the method or the use of a gene signature for determining a predisposition to develop obesity comprises determining an expression level of GLUL, SFRP4, ITIH5, GPC1, VGLL3 , C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, and PAMR1. In a preferred aspect thereof, underexpression of PHB2, SFXN2, IRS1, AACS, MTMR4, AATF, SLC25A38, DNAJB9, HOXA5, NDUFS2, BAG4, NCBP1, DGAT1, and SIK3 and overexpression of PCOLCE2, LHFPL2, TUBA1A, S100A11, ADAP2, SYNPO2, HEBP1, COL6A1, FADS3, MAN1B1, PID1, COL6A2, PMEPA1, HTRA3, RNASEL, and PAMR1 are indicative of a predisposition to develop obesity in said subject.

In an aspect, the method or the use of a gene signature for determining a predisposition to develop obesity comprises determining an expression level of GLUL, SFRP4, ITIH5, GPC1, VGLL3 , C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, and CERCAM. In a preferred aspect thereof, underexpression of PHB2, SFXN2, IRS1, AACS, MTMR4, AATF, SLC25A38, DNAJB9, HOXA5, NDUFS2, BAG4, NCBP1, DGAT1, SIK3, PPP1R15B, and NT5DC1 and overexpression of PCOLCE2, LHFPL2, TUBA1A, S100A11, ADAP2, SYNPO2, HEBP1, COL6A1, FADS3, MAN1B1, PID1, COL6A2, PMEPA1, HTRA3, RNASEL, PAMR1, PAM, STAT6, MAN2B1, and CERCAM are indicative of a predisposition to develop obesity in said subject.

In an aspect, the method or the use of a gene signature for determining a predisposition to develop obesity comprises determining an expression level of GLUL, SFRP4, ITIH5, GPC1, VGLL3 , C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, and SULF2. In a preferred aspect thereof, underexpression of PHB2, SFXN2, IRS1, AACS, MTMR4, AATF, SLC25A38, DNAJB9, HOXA5, NDUFS2, BAG4, NCBP1, DGAT1, SIK3, PPP1R15B, NT5DC1, SLC25A34, PPP1R1A, SLC27A1, TST, PDSS1, and LARP1 and over-expression of PCOLCE2, LHFPL2, TUBA1A, S100A11, ADAP2, SYNPO2, HEBP1, COL6A1, FADS3, MAN1B1, PID1, COL6A2, PMEPA1, HTRA3, RNASEL, PAMR1, PAM, STAT6, MAN2B1, CERCAM, ANXA1, COL18A1, ANKRD29, METRN, MAN2B2, IAH1, RTN2, FLOT1, and SULF2 are indicative of a predisposition to develop obesity in said subject.

In an aspect, the method or the use of a gene signature for determining a predisposition to develop obesity comprises determining an expression level of GLUL, SFRP4, ITIH5, GPC1, VGLL3 , C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, and LRRC20. In a preferred aspect thereof, underexpression of PHB2, SFXN2, IRS1, AACS, MTMR4, AATF, SLC25A38, DNAJB9, HOXA5, NDUFS2, BAG4, NCBP1, DGAT1, SIK3, PPP1R15B, NT5DC1, SLC25A34, PPP1R1A, SLC27A1, TST, PDSS1, LARP1, CSTF1, GDE1, UBLCP1, NUP54, ATAD3A, LCMT2, and BRWD1 and overexpression of PCOLCE2, LHFPL2, TUBA1A, S100A11, ADAP2, SYNPO2, HEBP1, COL6A1, FADS3, MAN1B1, PID1, COL6A2, PMEPA1, HTRA3, RNASEL, PAMR1, PAM, STAT6, MAN2B1, CERCAM, ANXA1, COL18A1, ANKRD29, METRN, MAN2B2, IAH1, RTN2, FLOT1, SULF2, HIP1, REEPS, and LRRC20 are indicative of a predisposition to develop obesity in said subject.

In an aspect, the method or the use of a gene signature for determining a predisposition to develop obesity comprises determining an expression level of GLUL, SFRP4, ITIH5, GPC1, VGLL3 , C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, and ANXA2. In a preferred aspect thereof, underexpression of PHB2, SFXN2, IRS1, AACS, MTMR4, AATF, SLC25A38, DNAJB9, HOXA5, NDUFS2, BAG4, NCBP1, DGAT1, SIK3, PPP1R15B, NT5DC1, SLC25A34, PPP1R1A, SLC27A1, TST, PDSS1, LARP1, CSTF1, GDE1, UBLCP1, NUP54, ATAD3A, LCMT2, BRWD1, LSM1, CSE1L, SLTM, JTB, HSPA9, RARRES1, and INTS4 and overexpression of PCOLCE2, LHFPL2, TUBA1A, S100A11, ADAP2, SYNPO2, HEBP1, COL6A1, FADS3, MAN1B1, PID1, COL6A2, PMEPA1, HTRA3, RNASEL, PAMR1, PAM, STAT6, MAN2B1, CERCAM, ANXA1, COL18A1, ANKRD29, METRN, MAN2B2, IAH1, RTN2, FLOT1, SULF2, HIP1, REEP5, LRRC20, PGM2L1, GPX7, MAP1LC3A, ARHGEF6, RSU1, AHNAK, SPATA6, PPFIA3, LGALS1, and ANXA2 are indicative of a predisposition to develop obesity in said subject.

In an aspect, the method or the use of a gene signature for determining a predisposition to develop obesity comprises determining an expression level of GLUL, SFRP4, ITIH5, GPC1, VGLL3 , C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, ANXA2, SMPDL3A, RBBP6, EYA2, ARL4A, PALMD, EPS15, TPCN1, PRELP, FXN, AP1S1, PLCE1, LEO1, PCBP1, SNTA1, MDFIC, ANKRD39, and NFIL3. In a preferred aspect thereof, underexpression of PHB2, SFXN2, IRS1, AACS, MTMR4, AATF, SLC25A38, DNAJB9, HOXA5, NDUFS2, BAG4, NCBP1, DGAT1, SIK3, PPP1R15B, NT5DC1, SLC25A34, PPP1R1A, SLC27A1, TST, PDSS1, LARP1, CSTF1, GDE1, UBLCP1, NUP54, ATAD3A, LCMT2, BRWD1, LSM1, CSE1L, SLTM, JTB, HSPA9, RARRES1, INTS4, RBBP6, FXN, LEO1, PCBP1, and NFIL3 and overexpression of PCOLCE2, LHFPL2, TUBA1A, S100A11, ADAP2, SYNPO2, HEBP1, COL6A1, FADS3, MAN1B1, PID1, COL6A2, PMEPA1, HTRA3, RNASEL, PAMR1, PAM, STAT6, MAN2B1, CERCAM, ANXA1, COL18A1, ANKRD29, METRN, MAN2B2, IAH1, RTN2, FLOT1, SULF2, HIP1, REEP5, LRRC20, PGM2L1, GPX7, MAP1LC3A, ARHGEF6, RSU1, AHNAK, SPATA6, PPFIA3, LGALS1, ANXA2, SMPDL3A, EYA2, ARL4A, PALMD, EPS15, TPCN1, PRELP, AP1S1, PLCE1, SNTA1, MDFIC, and ANKRD39 are indicative of a predisposition to develop obesity in said subject.

In an aspect, the method or the use of a gene signature for prediction of a predisposition to obesity comprises determining an expression level of GLUL, SFRP4, ITIH5, GPC1, VGLL3 , C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, ANXA2, SMPDL3A, RBBP6, EYA2, ARL4A, PALMD, EPS15, TPCN1, PRELP, FXN, AP1S1, PLCE1, LEO1, PCBP1, SNTA1, MDFIC, ANKRD39, NFIL3, METTL16, PPP1CC, SERPINF1, DIS3, STIM2, EMG1, CPT1A, and EHHADH. In a preferred aspect thereof, underexpression of PHB2, SFXN2, IRS1, AACS, MTMR4, AATF, SLC25A38, DNAJB9, HOXA5, NDUFS2, BAG4, NCBP1, DGAT1, SIK3, PPP1R15B, NT5DC1, SLC25A34, PPP1R1A, SLC27A1, TST, PDSS1, LARP1, CSTF1, GDE1, UBLCP1, NUP54, ATAD3A, LCMT2, BRWD1, LSM1, CSE1L, SLTM, JTB, HSPA9, RARRES1, INTS4, RBBP6, FXN, LEO1, PCBP1, NFIL3, METTL16, PPP1CC, DIS3, EMG1, and EHHADH and overexpression of PCOLCE2, LHFPL2, TUBA1A, S100A11, ADAP2, SYNPO2, HEBP1, COL6A1, FADS3, MAN1B1, PID1, COL6A2, PMEPA1, HTRA3, RNASEL, PAMR1, PAM, STAT6, MAN2B1, CERCAM, ANXA1, COL18A1, ANKRD29, METRN, MAN2B2, IAH1, RTN2, FLOT1, SULF2, HIP1, REEP5, LRRC20, PGM2L1, GPX7, MAP1LC3A, ARHGEF6, RSU1, AHNAK, SPATA6, PPFIA3, LGALS1, ANXA2, SMPDL3A, EYA2, ARL4A, PALMD, EPS15, TPCN1, PRELP, AP1S1, PLCE1, SNTA1, MDFIC, ANKRD39, SERPINF1, STIM2, and CPT1A are indicative of a predisposition to develop obesity in said subject.

The methods and uses of a gene signature for determining a predisposition to obesity described above can further be used for selecting a subject as susceptible to benefit from a preventive action against obesity.

The present invention relates to a method for monitoring a subject comprises predicting if the subject has a predisposition to develop obesity by the method of the invention, and selecting the subject having said predisposition as susceptible to benefit from a preventive action against obesity. Optionally, it may further comprise applying obesity prevention measures to the selected subject. For instance, such measures can include routinely measuring BMI, counseling on healthy eating and activity, reducing sedentary, appropriate follow-up laboratory tests, decreasing stress, increasing sleep, and administering weight loss medication, specially to an overweight subject.

The determination of the amounts or concentrations of proteins or mRNA encoded by the genes of the gene signature may comprise contacting the sample with selective reagents such as probes, primers or ligands, and thereby detecting the presence, or measuring the amount, of proteins or nucleic acids of interest originally in the sample.

The proteins or peptides can be assayed for by any one of the technologies well-known in the art, for instance by an immunoassay, by immunohistochemistry, by semi-quantitative Western-blot, by antibody arrays or by mass spectrometry.

Preferably, the expression level of the selected genes can be determined by measuring the amounts and/or concentrations of RNA, in particular mRNA, or DNA, in particular cDNA prepared from mRNA, in the biological sample using a variety of techniques well-known by the person skilled in art.

The expression level may be determined as a relative expression level (mRNA or protein). When mRNA is measured, the amounts of mRNA can be normalized to allow for better comparison between samples, using metrics such as reads per million mapped reads (RPM), reads per kilo base per million mapped reads (RPKM), transcript per million (TPM) or fragments per kilo base per million mapped fragments (FPKM).

The methods may comprise one or more of isolation of nucleic acids from the biological sample, purification of the nucleic acids, reverse transcription of RNA, and/or nucleic acid amplification.

In a preferred aspect, the expression level may be determined by determining the quantity of mRNA. Methods for determining the quantity of mRNA are well known in the art. For example, the nucleic acid contained in the samples (e.g., cell or tissue prepared from the individual) is first extracted according to standard methods, for example using lytic enzymes or chemical solutions or extracted by nucleic-acid-binding resins following the manufacturer's instructions. These nucleic acids may be frozen to be stored before use.

The mRNA or fragments thereof can be assayed for by any one of the technologies well-known in the art, for instance by quantitative or real-time reverse transcription polymerase chain reaction (RT-PCR or RT-qPCR), Q-beta replicase amplification, ligase chain reaction, signal amplification (Ampliprobe), light cycling, differential display, transcription-mediated amplification (TMA), NASBA (Nucleic Acid Based Amplification), Northern blot, Serial Analysis of Gene Expression (SAGE), DNA microarray, tiling array, RNA sequencing (RNA-Seq), DNA sequencing, MassArray analysis, digital PCR, *in situ* hybridization, or MALDI-TOF mass spectrometry.

The expression of certain genes known as "housekeeping genes", "reference genes", or "control genes" may also be determined in the biological sample as a means of ensuring the veracity of the expression profile. The reference genes or control genes have a stable expression in a particular tissue. Alternatively, such genes can be genes that are consistently expressed in many tissue types and thus are useful to normalize gene expression profiles. Determining the expression of housekeeping genes, reference genes, or control genes in parallel with the plurality of genes, may, for example, provide further assurance that the techniques used for determination of the gene expression profile are working properly. Appropriate housekeeping genes (also referred to herein as reference genes and control genes) can be readily selected by the skilled person.

When RT-PCR or RT-qPCR is used, it is usually performed using an internal reference. The ideal internal reference are genes expressed at a constant level among different samples, and whose expression is unaffected by the state or condition of the subject - for example, it is unaffected by whether or not the subject has a predisposition to obesity. RNAs most frequently used to normalize patterns of gene expression are mRNAs for housekeeping genes, for example glyceraldehyde-3-phosphate-dehydrogenase (GAPDH) and beta-actin.

When using RNA-Seq, differential expression, e.g. overexpression or underexpression of a gene when compared to a reference, can be computed using softwares and workflows known of the person skilled in the art, such as edgeR, DESeq2, limma. These packages and softwares are only given for illustrative purposes and a great number of software, packages and other bioinformatics tools can be used by the person skilled in the art.

In a particular aspect, the expression level of the markers or genes is compared to a reference expression level, for instance the expression level of the genes obtained in an individual, or a group of individuals, that are believed not to have predisposition to obesity. For instance, these individuals can by lean subjects, preferably without a predisposition to obesity or without family history of obesity. When a level has become well established for a particular population (control population or population having a predisposition to develop obesity), detection or quantification data can be directly compared with the known levels, called a reference level. Accordingly, the expression level can be an absolute value (e.g., a number of copies) for each gene. This value is compared to a threshold level of expression, thereby determining if a gene is overexpressed or underexpressed in the biological sample. The absolute value can be determined by digital PCR or with a plasmid including a particular number of copies.

The reference for expression of a gene may also be found in databases of gene expression data in individuals that do not exhibit obesity. An internal reference can also be used, for example using genes whose expression is unaffected by the state or condition of the subject - for example, it is unaffected by whether or not the subject has a predisposition to obesity. The level of expression of genes of this internal reference can be measured and compared to the level of expression of genes of the gene expression signatures of the invention.

**Kits**

Another object of the invention relates to a kit for the prediction or determination of a predisposition to obesity based on the use of the signatures of the invention. The present invention also relates to a kit comprising analysis means for detecting and/or quantifying in a biological sample, for example an adipose tissue biopsy sample, the combination of the level expression of genes of the gene signatures according the present invention and the use of the kit for determining if a subject has a predisposition to develop obesity. The kit may also be used to select a subject susceptible to benefit from a preventive action against obesity.

The means could be for instance gene specific reagents capable of detecting an expression product, such as primers and/or probe specific for a gene belonging of the gene signature. Optionally, the kit may comprise a microarray suitable for detecting and/or quantifying the expression of the genes of the gene signature.

Optionally, the kit or microarray is not suitable for detecting and/or quantifying the expression of more than 120, 130, 140, 150, 200, 250 or 300 genes.

For instance, the kit comprises means suitable for determining the expression levels of at least a gene signature comprising GLUL, SFRP4, ITIH5, GPC1, VGLL3 and C1QTNF1. Optionally, the kit can be primers and/or probe specific to GLUL, SFRP4, ITIH5, GPC1, VGLL3 and C1QTNF1.

Particularly, the kit comprises:

(i) at least one probe specific to mRNA or cDNA of each gene of the group consisting of GLUL, SFRP4, ITIH5, GPC1, VGLL3 and C1QTNF1, and, optionally, means for detecting the hybridization of said probe; and/or
(ii) at least one nucleic acid primer pair specific to each gene of the group consisting of GLUL, SFRP4, ITIH5, GPC1, VGLL3 and C1QTNF1 and, optionally, means for amplifying and/or detecting said mRNA or cDNA; and,
(iii) optionally, a leaflet providing guidelines to use such a kit and method to evaluate the expression level of such biomarkers.

The kit of the invention may further comprise analysis means for detecting and/or quantifying in a sample the expression level of 1, 2, 3, 4, 5, 6, 7, 8, 9, 10, 15, 20, 25, 30, 35, 40, 45, 50, 55, 60 or 65 additional genes selected from the group consisting of PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, ANXA2, SMPDL3A, RBBP6, EYA2, ARL4A, PALMD, EPS15, TPCN1, PRELP, FXN, AP1S1, PLCE1, LEO1, PCBP1, SNTA1, MDFIC, ANKRD39, NFIL3, METTL16, PPP1CC, SERPINF1, DIS3, STIM2, EMG1, CPT1A, and EHHADH.

Optionally, the kit of the invention may comprise

(i) at least one probe specific to mRNA or cDNA of each gene of one of the following groups, and, optionally, means for detecting the hybridization of said probes; and/or
(i) at least one probe specific to mRNA or cDNA of each gene of one of the following groups, and, optionally, means for detecting the hybridization of said probe; and/or
(iii) optionally, a leaflet providing guidelines to use such a kit and method to evaluate the expression level of such biomarkers,

wherein the groups consist of:

a) the genes GLUL, SFRP4, ITIH5, GPC1, VGLL3, C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, and AATF;
b) the genes GLUL, SFRP4, ITIH5, GPC1, VGLL3, C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, and NCBP1;
c) the genes GLUL, SFRP4, ITIH5, GPC1, VGLL3, C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, and PAMR1;
d) the genes GLUL, SFRP4, ITIH5, GPC1, VGLL3, C1QTNF1, GLUL, SFRP4, ITIH5, GPC1, VGLL3, C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, and CERCAM;

e) the genes GLUL, SFRP4, ITIH5, GPC1, VGLL3, C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, and SULF2;

f) the genes GLUL, SFRP4, ITIH5, GPC1, VGLL3, C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, and LRRC20;

g) the genes GLUL, SFRP4, ITIH5, GPC1, VGLL3, C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, and ANXA2;

h) the genes GLUL, SFRP4, ITIH5, GPC1, VGLL3, C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, ANXA2, SMPDL3A, RBBP6, EYA2, ARL4A, PALMD, EPS15, TPCN1, PRELP, FXN, AP1S1, PLCE1, LEO1, PCBP1, SNTA1, MDFIC, ANKRD39, and NFIL3; and

i) the genes GLUL, SFRP4, ITIH5, GPC1, VGLL3, C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, ANXA2, SMPDL3A, RBBP6, EYA2, ARL4A, PALMD, EPS15, TPCN1, PRELP, FXN, AP1S1, PLCE1, LEO1, PCBP1, SNTA1, MDFIC, ANKRD39, NFIL3, METTL16, PPP1CC, SERPINF1, DIS3, STIM2, EMG1, CPT1A, and EHHADH.

The kits of this invention are in suitable packaging. Suitable packaging includes, but is not limited to, vials, bottles, jars, flexible packaging (e.g., sealed Mylar or plastic bags), and the like. Instructions supplied in the kits of the invention are typically written instructions on a label or package insert (e.g., a paper sheet included in the kit in the form of a leaflet or instruction manual). In some aspects, the kit can comprise instructions for use in accordance with any of the methods described herein.

Finally, the present invention relates to the use of a kit as disclosed herein for determining if a subject has a predisposition to develop obesity.

*Table A: Gene description*

| Gene | Gene ID | name |
|------|---------|------|
| AACS | 78894 | acetoacetyl-CoA synthetase |
| AATF | 56321 | apoptosis antagonizing transcription factor |
| ADAP2 | 216991 | ArfGAP with dual PH domains 2 |
| AHNAK | 66395 | AHNAK nucleoprotein |
| ANKRD29 | 225187 | ankyrin repeat domain 29 |
| ANKRD39 | 109346 | ankyrin repeat domain 39 |
| ANXA1 | 16952 | annexin A1 |
| ANXA2 | 12306 | annexin A2 |
| AP1S1 | 11769 | adaptor protein complex AP-1, sigma 1 |
| ARHGEF6 | 73341 | Rac/Cdc42 guanine nucleotide exchange factor 6 |

(continued)

| Gene | Gene ID | name |
|---|---|---|
| ARL4A | 11861 | ADP-ribosylation factor-like 4A |
| ATAD3A | 108888 | ATPase family, AAA domain containing 3A |
| BAG4 | 67384 | BCL2-associated athanogene 4 |
| BRWD1 | 93871 | bromodomain and WD repeat domain containing 1 |
| C1QTNF1 | 56745 | C1q and tumor necrosis factor related protein 1 |
| CERCAM | 99151 | cerebral endothelial cell adhesion molecule |
| COL18A1 | 12822 | collagen, type XVIII, alpha 1 |
| COL6A1 | 12833 | collagen, type VI, alpha 1 |
| COL6A2 | 12834 | collagen, type VI, alpha 2 |
| CPT1A | 12894 | carnitine palmitoyltransferase 1a, liver |
| CSE1L | 110750 | chromosome segregation 1 like |
| CSTF1 | 67337 | cleavage stimulation factor, 3' pre-RNA, subunit 1 |
| OGAT1 | 13350 | diacylglycerol O-acyltransferase 1 |
| DIS3 | 72662 | DIS3 homolog, exosome endoribonuclease and 3'-5' exoribonuclease |
| DNAJB9 | 27362 | DnaJ heat shock protein family (Hsp40) member B9 |
| EHHADH | 74147 | enoyl-Coenzyme A, hydratase/3-hydroxyacyl Coenzyme A dehydrogenase |
| EMG1 | 14791 | EMG1 N1-specific pseudouridine methyltransferase |
| EPS15 | 13858 | epidermal growth factor receptor pathway substrate 15 |
| EYA2 | 14049 | EYA transcriptional coactivator and phosphatase 2 |
| FADS3 | 60527 | fatty acid desaturase 3 |
| FLOT1 | 14251 | flotillin 1 |
| FXN | 14297 | frataxin |
| GDE1 | 56209 | glycerophosphodiester phosphodiesterase 1 |
| GLUL | 14645 | glutamate-ammonia ligase |
| GPC1 | 14733 | glypican 1 |
| GPX7 | 67305 | glutathione peroxidase 7 |
| HEBP1 | 15199 | heme binding protein 1 |
| HIP1 | 215114 | huntingtin interacting protein 1 |
| HOXA5 | 15402 | homeobox A5 |
| HSPA9 | 15526 | heat shock protein 9 |
| HTRA3 | 78558 | HtrA serine peptidase 3 |
| IAH1 | 67732 | isoamyl acetate-hydrolyzing esterase 1 homolog |
| INTS4 | 101861 | integrator complex subunit 4 |
| IRS1 | 16367 | insulin receptor substrate 1 |
| ITIH5 | 209378 | inter-alpha-trypsin inhibitor, heavy chain 5 |
| JTB | 23922 | jumping translocation breakpoint |
| LARP1 | 73158 | La ribonucleoprotein 1, translational regulator |
| LCMT2 | 329504 | leucine carboxyl methyltransferase 2 |
| LEO1 | 235497 | Leo1, Paf1/RNA polymerase II complex component |
| LGALS1 | 16852 | lectin, galactose binding, soluble 1 |
| LHFPL2 | 218454 | lipoma HMGIC fusion partner-like 2 |
| LRRC20 | 216011 | leucine rich repeat containing 20 |
| LSM1 | 67207 | LSM1 homolog, mRNA degradation associated |
| MAN1B1 | 227619 | mannosidase, alpha, class 1B, member 1 |
| MAN2B1 | 17159 | mannosidase 2, alpha B1 |
| MAN2B2 | 17160 | mannosidase 2, alpha B2 |
| MAP1LC3A | 66734 | microtubule-associated protein 1 light chain 3 alpha |
| MDFIC | 16543 | MyoD family inhibitor domain containing |
| METRN | 70083 | meteorin, glial cell differentiation regulator |
| METTL16 | 67493 | methyltransferase 16, N6-methyladenosine |

(continued)

| Gene | Gene ID | name |
|------|---------|------|
| MTMR4 | 170749 | myotubularin related protein 4 |
| PAM | 105689 | MYC binding protein 2, E3 ubiquitin protein ligase |
| NCBP1 | 433702 | nuclear cap binding protein subunit 1 |
| NDUFS2 | 226646 | NADH:ubiquinone oxidoreductase core subunit S2 |
| NFIL3 | 18030 | nuclear factor, interleukin 3, regulated |
| NT5DC1 | 319638 | 5'-nucleotidase domain containing 1 |
| NUP54 | 269113 | nucleoporin 54 |
| PALMD | 114301 | palmdelphin |
| PAMR1 | 210622 | peptidase domain containing associated with muscle regeneration 1 |
| PCBP1 | 23983 | poly(rC) binding protein 1 |
| PCOLCE2 | 76477 | procollagen C-endopeptidase enhancer 2 |
| POSS1 | 56075 | prenyl (solanesyl) diphosphate synthase, subunit 1 |
| PGM2L1 | 70974 | phosphoglucomutase 2-like 1 |
| PHB2 | 12034 | prohibitin 2 |
| PLCE1 | 74055 | phospholipase C, epsilon 1 |
| PMEPA1 | 65112 | prostate transmembrane protein, androgen induced 1 |
| PPFIA3 | 76787 | protein tyrosine phosphatase, receptor type, f polypeptide (PTPRF), interacting protein (liprin), alpha 3 |
| PPP1CC | 434233 | protein phosphatase 1 catalytic subunit gamma B |
| PPP1R15B | 108954 | protein phosphatase 1, regulatory subunit 15B |
| PPP1R1A | 58200 | protein phosphatase 1, regulatory inhibitor subunit 1A |
| PRELP | 116847 | proline arginine-rich end leucine-rich repeat |
| PID1 | 107422 | prior incubation determinant 1 |
| RARRES1 | 109222 | retinoic acid receptor responder (tazarotene induced) 1 |
| RBBP6 | 19647 | retinoblastoma binding protein 6, ubiquitin ligase |
| REEP5 | 13476 | receptor accessory protein 5 |
| RNASEL | 24014 | ribonuclease L (2', 5'-oligoisoadenylate synthetase-dependent) |
| RSU1 | 20163 | Ras suppressor protein 1 |
| RTN2 | 20167 | reticulon 2 (Z-band associated protein) |
| S100A11 | 20195 | S100 calcium binding protein A11 |
| SERPINF1 | 20317 | serine (or cysteine) peptidase inhibitor, clade F, member 1 |
| SFRP4 | 20379 | secreted frizzled-related protein 4 |
| SFXN2 | 94279 | sideroflexin 2 |
| SIK3 | 70661 | SIK family kinase 3 |
| SLC25A34 | 384071 | solute carrier family 25, member 34 |
| SLC25A38 | 208638 | solute carrier family 25, member 38 |
| SLC27A1 | 26457 | solute carrier family 27 (fatty acid transporter), member 1 |
| SLTM | 66660 | SAFB-like, transcription modulator |
| SMPDL3A | 57319 | sphingomyelin phosphodiesterase, acid-like 3A |
| SNTA1 | 20648 | syntrophin, acidic 1 |
| SPATA6 | 67946 | spermatogenesis associated 6 |
| STAT6 | 20852 | signal transducer and activator of transcription 6 |
| STIM2 | 116873 | stromal interaction molecule 2 |
| SULF2 | 72043 | sulfatase 2 |
| SYNPO2 | 118449 | synaptopodin 2 |
| TPCN1 | 252972 | two pore channel 1 |
| TST | 22117 | thiosulfate sulfurtransferase, mitochondrial |
| TUBA1A | 22142 | tubulin, alpha 1A |
| UBLCP1 | 79560 | ubiquitin-like domain containing CTD phosphatase 1 |
| VGLL3 | 73569 | vestigial like family member 3 |

The examples below illustrate the invention without limiting its scope.

**EXAMPLES**

Example 1: high variability of the proneness to diet-induced obesity (DIO) between individuals

In a first set of experiments, the inventors assessed the weight gain in responses to a nutritional challenge (High-Fat Diet or HFD) of a cohort (n=27) of inbred (i.e., genetically very close) adult mice presenting a uniform basal phenotype. The experimental design is indicated in Figure 1A. As shown in Figure 1B, at timepoint TO, the distribution of body weight and fat mass among the mice followed a normal distribution. However, after the 18-week HFD-challenge (T18), a wide range of sensitivities to diet-induced obesity (DIO) were observed, with the emergence of three distinct groups. Specifically, certain mice exhibited minimal weight gain and were thus classified as DIO-resistant (R), whereas others exhibited excessive weight gain and were considered prone to DIO (P). An intermediate group with intermediate weight gain was also identified (I).

To comprehensively consider all physical parameters including body weight, fat mass, and adiposity at T18, delta% body weight, delta% fat mass, and delta% adiposity, a Hierarchical Clustering Analysis (HCA) was conducted (Figure 1C). The results confirmed that three distinct groups of mice were present following the HFD-challenge. Based on this a posteriori HCA (Figure 1D), for the first group, referred to as Resistant (R), body weights at TO and T18 were not significantly different, with only slight increases in fat mass and adiposity. The second group, referred to as Intermediate (I), exhibited a significant increase in all three parameters between TO and T18. The extent of the increase was higher than observed for the R group. Finally, the third group, referred to as Prone (P), gained considerable body weight, fat mass, and adiposity, to significantly higher extents than the other groups. These animals were therefore considered highly susceptible to DIO. These results confirm that, within an ostensibly homogeneous population, some individuals are highly sensitive to DIO and are therefore prone to developing associated pathologies, whereas others are more resistant.

These observations indicated that prior to the HFD-challenge, at TO, none of the parameters measured (body weight, fat mass, and adiposity) could reliably predict an individual's response to a HFD. This conclusion was further supported by attempts to develop a predictive model using partial least squares discriminant analysis (PLS DA) based solely on the physical parameters measured at TO. In contrast, PLS-DA and permutation tests conducted on the T18 data confirmed that the three groups identified are indeed significantly different and can be discriminated based on their physical parameters.

Example 2: Identification of a set of genes as early predictive markers of predisposition to DIO

The inventors' main objective was to identify genes with an expression pattern correlating with predisposition to DIO. The inventors specifically focused their study on perigonadal white adipose tissue (pg WAT) due to its metabolic relevance and its ease of accessibility for biopsies. One week before the change in diet (T0) a pg WAT biopsy was taken. At the final time point (T18) following the 18 week HFD challenge, pg WAT was taken at sacrifice.

Based on the clustering shown in Figure 1C, the pg WAT gene expression profiles were compared for the Resistant, Prone, and Intermediate groups at TO and T18. The workflow used for this analysis is illustrated in Figure 2A. First, differential expression analysis was conducted on the microarray gene expression data (Array Express E-MTAB-13877 and E-MTAB-13878) using the Limma package. A total of 2356 mRNA species were differentially expressed at TO, and 1020 mRNA species were differentially expressed at T18 according to the group factor (I, R, or P). This preliminary statistical analysis was later used to refine the gene list. Second, PLS DA was performed on the same dataset to identify variables that could effectively identify individuals belonging to the three groups at each time point. To assess the reliability of the models obtained and ensure they were not affected by overfitting or excessive randomness, permutation tests were conducted by randomly permuting (500 times) the individuals in the initial dataset. The error rates obtained with all the permuted data were higher than with the original dataset, thus the validity of the PLS DA models obtained was confirmed. To assess the contribution of each variable to the models, Variable of Importance in Projection (VIP) scores were calculated. A threshold VIP of $\geq 1.5$ was applied, as it represents a robust criterion to select variables of interest. Following application of this threshold, the inventors retained 3387 mRNA species at TO and 1746 mRNA species at T18 as potential variables of interest in the analysis (Figure 2B).

Principal Component Analysis (PCA) was then applied to these data - the set of 3387 mRNA species at TO and 1746 mRNA species at T18. The aim was to evaluate the ability of these mRNA species to effectively classified mice into their original group assignments. The results of the PCA analysis, as shown in Figure 2C, demonstrated that these selected mRNA species had a significant discriminatory capacity, particularly for mice in group P. Thus, these mRNA species could be useful indicators to identify individuals with a predisposition to DIO.

Example 3: impact of the mother's nutritional status on the metabolic fate of offspring

Considering that in the model depicted above, the animals are genetically very close, the origine of the differences observed in susceptibility to obesity could potentially be attributed to epigenetic factors. In line with the principles of the Developmental Origins of Health and Disease (DOHaD) concept, susceptibility to obesity could be influenced by challenges experienced during perinatal life, especially depending on the mother's nutritional status.

The inventors assessed the effects of the type and timing of maternal nutritional stress on predisposition to DIO among offspring. As shown in Figure 3A, a control group of males born to mothers fed a control diet (F1 CD, group A) was compared to the following experimental groups: males born to mothers fed a Low-Protein Diet (LPD) during both gestation and lactation (F1 LPD, group B), males born to mothers fed a LPD during gestation only (F1 LPD CD, group C), males born to mothers fed a HFD during lactation (F1-CD-HFD, group D), and males born to mothers fed a LPD during lactation only (F1 CD LPD, group E). After weaning, all male offspring from these groups were fed a control diet. Various biological parameters were measured in adult male offspring from the five groups. Figure 3B (top-left) shows the body weight measured from 10 days and throughout life, up to 16 months. At 5 months, the weight of the animals is presented Figure 3B (bottom). This analysis reveals that males from group C showed no difference in body weight compared to the control group A, whereas males from groups B and E had lower body weight, and males from group D had higher body weight. Similar trends were observed when considering the fat mass of the animals (Figure 3B-bottom). It is interesting to note that maternal nutritional stress had a greater impact on fat mass than on lean mass, although both were affected. Representative photographs of animals from extreme groups at 10 days and 2 months of age further illustrate the considerable differences in body weight linked to maternal nutritional status (Figure 3B, top-right). Overall, these findings highlight the major impact of the type and timing of maternal nutritional stress during the perinatal period on the developmental trajectory of offspring. In subsequent investigations, the inventors specifically focused on the control group (A) and the two experimental groups that displayed the highest and lowest body weight - groups D and B, respectively. To determine the predisposition of the offspring from each group toward obesity, young adult F1 animals (5 months old) were fed a HFD for 7 months, according to the experimental protocol presented in Figure 3C. As shown Figure 3D, control animals (group A) gained two-fold more weight when fed a HFD compared to a control diet (medium effect size; Cohen's d = 0.54). In contrast, group D animals gained 4 times more weight when fed a HFD compared to a control diet (large effect size; Cohen's d = 0.92). Interestingly, group B animals appeared to be protected against HFD-induced obesity, gaining only 1.5 times more weight when fed a HFD rather than a control diet.

An oral glucose tolerance test (OGTT) was conducted at the end of the 7-month HFD-challenge. As shown in Figure 3E, the area under the curve for group D animals was the highest among the groups, suggesting a tendency toward insulin resistance. No differences were observed between the groups before the HFD-challenge (data not shown). Moreover, blood glucose levels were consistently higher in group D animals.

In summary, these findings demonstrate that group B animals are resistant to DIO, whereas group D animals are prone to DIO, indicating that maternal nutritional stress can produce a metabolic imprint in the offspring that significantly influences their susceptibility to metabolic disorders. Based on these results, groups B and D could be seen as equivalent to groups R and P identified in the previous model, but obtained by induction rather than stochastically. These results reinforce the role of perinatal life as a major determinant in an individual's metabolic fate.

Example 4: identification of a set of genes whose expression correlates with predisposition to DIO in a nutritional programming model

RNA seq analysis was conducted on pg WAT samples from 5 month old animals from groups A, B, and D (Array Express E-MTAB-13879). The analysis workflow is illustrated in Figure 4A. First, EdgeR-based differential expression analysis was performed on the RNA seq data, resulting in the identification of 2145 mRNA species that displayed significant differential expression for the group factor (A, B, D). This preliminary statistical analysis was later used to refine the list of genes. The same dataset was then subjected to PLS DA to identify variables capable of distinguishing individuals between the three groups. As previously, to assess the reliability of the model obtained and to determine if overfitting or excessive randomness were problematic, permutation tests were conducted. The error rates obtained with the permuted data were higher than those obtained with the original dataset, confirming the validity of the model. The contribution of each variable to the model was assessed by calculating the VIP. In total, 1889 mRNA species exhibited a Variable Importance in Projection (VIP) ≥ 1.5. These corresponded to 1873 unique gene symbols, which were selected and considered as potential variables of interest in the analysis (Figure 4B).

Using PCA (Figure 4C), the inventors investigated how well these 1889 mRNA species classify mice in their original groups. The results revealed that these mRNA species were effective indicators of membership of either the B or D groups. Taken together, these results demonstrate that maternal nutritional stress can produce a metabolic imprint in offspring, making them more or less prone to DIO and related conditions. This imprint is associated with a specific gene expression pattern, which could potentially serve as a means to determine susceptibility to DIO.

Example 5: a set of predictive genes has discriminant potential regarding predisposition to DIO

To identify a robust set of predictive genes, the inventors compared the gene signatures obtained from the PLS DA applied to their two models, innate variability model and nutritional programming model (Figure 5A). This comparison led to the identification of 201 common discriminant genes. However, upon further analysis, four of these genes (despite having a VIP $\geq 1.5$ for the 3 analyses described above) were found to have an adjusted q-value (as calculated above using ANOVA) exceeding 0.05 across all three datasets (ABD, TO, T18, where ABD is the dataset resulting from the analysis of groups A, B and D in the nutritional programming model). Consequently, these genes were excluded from the analysis. As a result, a final set of 197 genes was selected for further investigation.

To assess the discriminatory potential of these 197 genes before the HFD challenge, the inventors performed PCA on TO data from model 1 (diet-induced obesity). The results shown in Figure 5B demonstrate that, based on the expression patterns of these 197 genes in experimental model 1 at TO, it is indeed possible to distinguish obesity prone animals from the animals in the other groups. This finding highlights the potential usefulness of these genes for the reliable identification of individuals with a predisposition to DIO.

Example 6: Identification of candidate genes for the prediction of predisposition to develop obesity

The inventors assessed if the human orthologs of the 197 candidate genes identified in their two mouse models above could also be considered as good candidates to be predictive of susceptibility to obesity in humans.

To assess whether the 197 genes identified in mice might also be discriminative in humans, the inventors combined their three mouse datasets, described above, with four independent external datasets derived from previous studies on humans. To closely match human data, the mouse data sets used were composed of groups A and D from the nutritional programing model and group I and P from the innate variability model at TO and T18. Indeed, the goal was to identify individuals who are sensitive compared to the general population, rather than compared to a specific, resistant population.

Briefly, each human dataset used is composed of two groups (lean and obese) for which transcriptomic analysis (either through microarray or RNA seq) was performed on WAT (either subcutaneous or omental), from both male and female individuals. For the inventors' analysis, they first analyzed all datasets to identify which of the 197 mouse genes are present in all human datasets. Following this alignment, 143 genes remained for which expression data was available for a total of 141 individual samples (either mice or human) from the seven integrated datasets.

To deal with the heterogeneity due to the use of data from various sources, horizontal data integration was performed using the R mixOmics package MINT methods (P integration). In the MINT toolbox, the inventors used mint.plsda and mint.splsda to build two successive classification models for discriminative and predictive purposes. A first basic 5 component classification model, including the 143 common variables with a two group outcome (lean vs obese) was produced, and its performance assessed. A first graphical output projected all the samples onto a unique space, with a good separation of samples based on their group, especially along the axis corresponding to component 1 (which retains 17% of the overall variance). A second visualization allowed the partial projection of samples, one study after another. The results confirmed that the group discrimination is adequate for all seven studies, whether involving mice or humans, especially along the axis for component 1. This analysis also assigned weights (or loading) to the different variables depending on their relative contributions to the model (Figure 6A and Table 1). Based on this loading, the candidate genes could be hierarchically organized. The 23 most predictive genes - those with the highest absolute values, at the top of the hierarchy - are presented in Table 1.

*Table 1: top 23 of the genes according to their absolute loading value (absolute loading value > 0.12), selected arbitrarily, for illustration purpose, on the basis of the break observed in the distribution graph (Figure 6A).*

| Top 23 genes | Loadings Comp1 |
| --- | --- |
| GLUL | -0.188558551 |
| SFRP4 | 0.179390332 |
| ITIH5 | 0.174610482 |
| GPC1 | 0.16370043 |
| VGLL3 | 0.155168921 |
| C1QTNF1 | 0.150857025 |
| PCOLCE2 | 0.147788693 |
| PHB2 | -0.140598735 |

(continued)

| Top 23 genes | Loadings Comp1 |
|---|---|
| SFXN2 | -0.14003348 |
| LHFPL2 | 0.137810059 |
| TUBA1A | 0.136056774 |
| S100A11 | 0.134771492 |
| IRS1 | -0.134408379 |
| ADAP2 | 0.132512035 |
| SYNPO2 | 0.132454244 |
| AACS | -0.131029654 |
| MTM R4 | -0.128655794 |
| HEBP1 | 0.128615275 |
| COL6A1 | 0.128120813 |
| FADS3 | 0.125542481 |
| MAN1B1 | 0.125520733 |
| PID1 | 0.122539323 |
| AATF | -0.121338582 |

The performance of this first prediction model when seeking to distinguish between lean and obese individuals can be assessed in two ways: the first output is the ROC (Receiver Operating Characteristic) curve and its AUC (Area Under Curve) which give a first clue as to the overall quality of the model (Figure 6B). With an AUC at 0.812 when focused on component 1, the model has very good capacity to predict the two phenotypes. This was confirmed by the second output, which corresponds to the diverse low error rates calculated by the "perf" functions and its LOGOCV algorithm (Leave one Group Out Cross Validation). This algorithm sequentially isolates each of the 7 datasets and uses it as an external set to be predicted. It returns overall error rates of these predictions that assess the model's predictive power. For the inventors' first model, they looked mainly at the BER (Balanced Error Rate) on component 1, the main variability axis containing most of their data. For this component, the BER value obtained - 0.178 - was satisfactory. Another parameter, namely the group error rate on component 1, gave a very satisfactory value of 0.10 for the prediction of the "obese" group, whereas a higher value of 0.254 was obtained for the "lean" group, indicating that this class is more difficult to assign (predict), probably due to the higher dispersion of its samples.

Secondly, an optimized model was generated using the same data and the sparse mint.splsda method. This optimization process involved determining the minimal number of (1) components, and (2) genes that can be retained in the model without affecting prediction rates. Following this optimization, only one component was retained in the model; it was associated with 109 of the initial 143 genes (Figure 6C) (see Table 2). Applying these two parameters reduced the overall error rates (from 0.1783 to 0.1306). In comparison to the first model outputs, the gene hierarchy remained the same, but with higher absolute loading values (for the first 34 genes). The quality assessments, from ROC curves (Figure 6D) or error rates, revealed a slightly better model with an AUC of 0.818, a BER at 0.131, and a group error rate of 0.16 for the lean group. The error rate for the obese group remained unchanged (0.1026).

Having identified 109 genes whose expression can be used as a signature for the prediction of obesity, the inventors assessed the possibility of reducing the number of genes necessary to predict obesity. In other words, the inventors sought to identify the best compromise between simplicity (number of genes) and model reliability. Using the Balanced Error Rates, they identified thresholds for the number of genes involved in the model at which the BER substantially shifts: 6, 23, 32, 36, 42, 57, 67, 84, and 101 genes. These shifts are shown by red arrows in Figure 6E, where the BER is on the y-axis and the AUC of the ROC (or AUROC) curve is on the x-axis. Commonly, models with an AUROC between 0.7 and 0.8 are considered acceptable, between 0.8 and 0.9 excellent, and above 0.9 outstanding (these thresholds are indicated by a vertical red dotted line in Figure 6E). The 6-genes model adjoins a model that would be acceptable, and seems to be the minimal model. The six genes in this model are GLUL, SFRP4, ITIH5, GPC1, VGLL3 and C1QTNF1. Based on the thresholds mentioned above, it is possible to define gene expression signatures, which are summarized in Table 2.

| Table 2: Genes signatures based on | Loadings | 6-genes signature | 23-genes signature | 32-genes signature | 36-genes signature | 42-genes signature | 57-genes signature | 67-genes signature | 84-genes signatures | 101-genes signature | 109-genes signature |
|---|---|---|---|---|---|---|---|---|---|---|---|
| GLUL | -0,18855855 | | | | | | | | | | |
| SFRP4 | 0,17939033 | | | | | | | | | | |
| ITIH5 | 0,17461048 | | | | | | | | | | |
| GPC1 | 0,16370043 | | | | | | | | | | |
| VGLL3 | 0,15516892 | | | | | | | | | | |
| C1QTNF1 | 0,15085703 | | | | | | | | | | |
| PCOLCE2 | 0,14778869 | | | | | | | | | | |
| PHB2 | -0,14059873 | | | | | | | | | | |
| SFXN2 | -0,14003348 | | | | | | | | | | |
| LHFPL2 | 0,13781006 | | | | | | | | | | |
| TUBA1A | 0,13605677 | | | | | | | | | | |
| S100A11 | 0,13477149 | | | | | | | | | | |
| IRS1 | -0,13440838 | | | | | | | | | | |
| ADAP2 | 0,13251204 | | | | | | | | | | |
| SYNPO2 | 0,13245424 | | | | | | | | | | |
| AACS | -0,13102965 | | | | | | | | | | |
| MTMR4 | -0,12865579 | | | | | | | | | | |
| HEBP1 | 0,12861527 | | | | | | | | | | |
| COL6A1 | 0,12812081 | | | | | | | | | | |
| FADS3 | 0,12554248 | | | | | | | | | | |
| MAN1B1 | 0,12552073 | | | | | | | | | | |
| PID1 | 0,12253932 | | | | | | | | | | |
| AATF | -0,12133858 | | | | | | | | | | |
| SLC25A38 | -0,11723131 | | | | | | | | | | |
| DNAJB9 | -0,11466628 | | | | | | | | | | |
| COL6A2 | 0,1137211 | | | | | | | | | | |
| HOXA5 | -0,11295416 | | | | | | | | | | |
| NDUFS2 | -0,11156748 | | | | | | | | | | |
| BAG4 | -0,11008999 | | | | | | | | | | |
| PMEPA1 | 0,10820982 | | | | | | | | | | |
| HTRA3 | 0,10801545 | | | | | | | | | | |
| NCBP1 | -0,10743581 | | | | | | | | | | |
| DGAT1 | -0,10640914 | | | | | | | | | | |
| RNASEL | 0,10484579 | | | | | | | | | | |
| SIK3 | -0,1043809 | | | | | | | | | | |
| PAMR1 | 0,10379691 | | | | | | | | | | |
| PAM | 0,10212853 | | | | | | | | | | |
| STAT6 | 0,10130359 | | | | | | | | | | |
| PPP1R15B | -0,09659165 | | | | | | | | | | |
| NT5DC1 | -0,09656249 | | | | | | | | | | |
| MAN2B1 | 0,09636924 | | | | | | | | | | |
| CERCAM | 0,09556356 | | | | | | | | | | |

| | | | | | | | | | | | | | |
|---|---|---|---|---|---|---|---|---|---|---|---|---|---|
| ANXA1 | 0,09548846 | | | | | | | | | | | | |
| COL18A1 | 0,09533412 | | | | | | | | | | | | |
| SLC25A34 | -0,09255902 | | | | | | | | | | | | |
| ANKRD29 | 0,09234283 | | | | | | | | | | | | |
| METRN | 0,09193841 | | | | | | | | | | | | |
| MAN2B2 | 0,09116594 | | | | | | | | | | | | |
| PPP1R1A | -0,08969894 | | | | | | | | | | | | |
| IAH1 | 0,0859614 | | | | | | | | | | | | |
| SLC27A1 | -0,08538298 | | | | | | | | | | | | |
| TST | -0,08500818 | | | | | | | | | | | | |
| RTN2 | 0,08369405 | | | | | | | | | | | | |
| FLOT1 | 0,08189796 | | | | | | | | | | | | |
| PDSS1 | -0,07889117 | | | | | | | | | | | | |
| LARP1 | -0,07861492 | | | | | | | | | | | | |
| SULF2 | 0,07613887 | | | | | | | | | | | | |
| CSTF1 | -0,07608891 | | | | | | | | | | | | |
| HIP1 | 0,07464091 | | | | | | | | | | | | |
| GDE1 | -0,07408471 | | | | | | | | | | | | |
| UBLCP1 | -0,07314621 | | | | | | | | | | | | |
| NUP54 | -0,07274233 | | | | | | | | | | | | |
| ATAD3A | -0,07249483 | | | | | | | | | | | | |
| LCMT2 | -0,07244745 | | | | | | | | | | | | |
| BRWD1 | -0,07242834 | | | | | | | | | | | | |
| REEP5 | 0,07197991 | | | | | | | | | | | | |
| LRRC20 | 0,07174907 | | | | | | | | | | | | |
| LSM1 | -0,07110892 | | | | | | | | | | | | |
| CSE1L | -0,07090017 | | | | | | | | | | | | |
| SLTM | -0,06985114 | | | | | | | | | | | | |
| PGM2L1 | 0,06956365 | | | | | | | | | | | | |
| GPX7 | 0,06939398 | | | | | | | | | | | | |
| MAP1LC3A | 0,069347 | | | | | | | | | | | | |
| ARHGEF6 | 0,06823155 | | | | | | | | | | | | |
| JTB | -0,06805003 | | | | | | | | | | | | |
| RSU1 | 0,06709595 | | | | | | | | | | | | |
| HSPA9 | -0,06660542 | | | | | | | | | | | | |
| AHNAK | 0,06635388 | | | | | | | | | | | | |
| SPATA6 | 0,06588633 | | | | | | | | | | | | |
| RARRES1 | -0,06531838 | | | | | | | | | | | | |
| PPFIA3 | 0,06255208 | | | | | | | | | | | | |
| INTS4 | -0,06209806 | | | | | | | | | | | | |
| LGALS1 | 0,060371 | | | | | | | | | | | | |
| ANXA2 | 0,05833826 | | | | | | | | | | | | |
| SMPDL3A | 0,05809712 | | | | | | | | | | | | |
| RBBP6 | -0,05796507 | | | | | | | | | | | | |
| EYA2 | 0,05584966 | | | | | | | | | | | | |
| ARL4A | 0,05529891 | | | | | | | | | | | | |

| | | | | | | | | | | | |
|---|---|---|---|---|---|---|---|---|---|---|---|
| PALMD | 0,05488775 | | | | | | | | | | |
| EPS15 | 0,05342317 | | | | | | | | | | |
| TPCN1 | 0,05275396 | | | | | | | | | | |
| PRELP | 0,05198961 | | | | | | | | | | |
| FXN | -0,05187903 | | | | | | | | | | |
| AP1S1 | 0,05055083 | | | | | | | | | | |
| PLCE1 | 0,04947214 | | | | | | | | | | |
| LEO1 | -0,04854881 | | | | | | | | | | |
| PCBP1 | -0,04852229 | | | | | | | | | | |
| SNTA1 | 0,04843475 | | | | | | | | | | |
| MDFIC | 0,04837746 | | | | | | | | | | |
| ANKRD39 | 0,04334989 | | | | | | | | | | |
| NFIL3 | -0,04090737 | | | | | | | | | | |
| METTL16 | -0,03904544 | | | | | | | | | | |
| PPP1CC | -0,0384094 | | | | | | | | | | |
| SERPINF1 | 0,0380973 | | | | | | | | | | |
| DIS3 | -0,0378772 | | | | | | | | | | |
| STIM2 | 0,03734838 | | | | | | | | | | |
| EMG1 | -0,03611308 | | | | | | | | | | |
| CPT1A | 0,03565794 | | | | | | | | | | |
| EHHADH | -0,03408591 | | | | | | | | | | |

.

Materials and methods

All experiments were conducted with the approval of the regional ethics committee (agreement no. D6334515) following the European Directive 2010/63/EU on the protection of vertebrate animals used for experimental and scientific purposes.

All BALB/c mice (strain BALB/cAnNRj) were purchased from Janvier-Labs (Le Genest-Saint-Isle, France) at 5-weeks of age. Upon arrival, they were housed in a controlled room (22 $\pm$ 2∘C, 60 $\pm$ 5% humidity, 12 h light/dark cycle, and light period starting at 8:00am), fed ad libitum a standard rodent diet (A03 from Safe, Augy, France), and given free access to water, until they reached the age required for experimental protocols described below.

*Innate variability model*

Twenty-seven 4-month-old BALB/c male mice were used. At TO, perigonadal WAT (pgWAT) biopsies from each mice were performed. One week after surgery, mice were fed an experimental High Fat Diet (HFD, Open Source Diet, Ref D12451) for 18 weeks after which they were sacrificed (T18) for removal of perigonadal WAT. The experimental plan is outlined in Figure 1A. Body weight and body composition were monitored at various stages. The initial number of animals (n=27) was determined based on previous experiments in order to be able to generate 3 groups of n >6 mice based on body weight changes following HFD challenge (either Resistant, Intermediate or Prone to DIO).

*Nutritional programming model*

Two-month-old virgin BALB/c female mice fed a chow diet ((A03; Safe, Augy, France) were mated with 2-month-old BALB/c male, isolated at vaginal plug detection and randomly transferred on Experimental Diet (CD: Control Diet, LPD: Low Protein Diet, HFD: High Fat Diet) as indicated figure 3A. At parturition, randomization was carried out as follows: dams and litters were transferred on experimental diet as indicated figure 3A. Litters of different sizes were obtained in each group of pregnant female. Since the litter size is important in the offspring life trajectory, the inventors' strategy was to erase all possible confounding factors coming from litter size and the allotting protocol was as follows: Mice from litter of 3 pups or less or from litter of more than 10 pups were excluded to minimize the variability of each parameter known to be impacted by litter size and thus only litters that have a total number of pups comprised between 4 and 10 were considered, to avoid extreme litter size. After weaning at 4 weeks of age, male offspring from each group were housed individually with free access to Control Diet (CD). To obviate any litter effects, animals used for each experiment were randomly chosen in different litters, and only a limited number of animals (n = 1 to 2) was used from each litter. The initial number of animal used was determined based on previous experiment in order to generate male offspring number > 8 per group.

CD and LPD (UPAE, Jouy-en-Josas, France) contain respectively 22% and 10% protein and composition are given in Jousse et al., 2011 (doi: 10.1096/fj.11-181792). HFD (High Fat Diet) contains 45% kCal from fat (Open Source Diet Ref D12451).

Body weight and body composition were monitored at various ages throughout life for all F1 male mouse groups. Part of F1 male mice of each group were sacrificed at 6 mo for further analyses and part of F1 male mice from groups A, B and D were submitted to a HFD-challenge from the age of 5 to 12-month.

*Body composition and metabolic parameters*

Individual mice were placed into restrain tube and inserted into the mouse EchoMRI-100 instrument (Echo Medical Systems LLC, Houston TX, USA) to determine both fat and lean mass (g). Total body weight was measured using a standard top-loading laboratory balance. Adiposity is expressed as a percentage of fat relative to total body weight.

Total cholesterol, HDL-cholesterol, triglycerides, and glucose concentration were measured in plasma of overnight starved adult male mice, using commercial enzymatic assays.

For oral glucose tolerance test (OGTT) mice were given an oral glucose bolus (2 g/kg) after a 7 hour fast. Blood was collected from the tail vein at different times. Glycemia was measured using the OneTouch glucometer (Lifescan Inc., Milpitas, CA).

*Tissue collection*

For peri-gonadal WAT biopsy, mice were placed on an heating-pad under isoflurane anesthesia using a nose cone. At the end of experiments, mice were euthanized by cervical dislocation under isoflurane anesthesia. In both cases, perigonadal WAT was collected prior to immediate freezing in liquid nitrogen and stored at -80 °C until analyses. Chirurgy and euthanasy were performed by alterning one mouse of each group in order to minimize potential confounders.

*Transcriptomic analysis*

• RNA-Seq gene expression studies

Prepared RNAs sample were sent to the sequencing facility (IGENSEQ) at the ICM Institute (Paris, France), and their quality and concentration were checked using bioanalyzer. Libraries were prepared using the "TruSeq Stranded mRNA" kit (ILLUMINA®), allowing the construction of a library of polyadenylated RNAs (mRNA and polyadenylated non-coding RNAs such as lncRNA) from total RNAs. The libraries were prepared following the manufacturer's recommendations and subsequently sequenced using the Nextseq 500 (ILLUMINA®) to obtain $2 \times 40$ million 75-base pair fragments per sample.

• Micro-array Gene Expression studies

Gene expression profiles were performed at the GeT-TRiX facility (GénoToul, Génopole Toulouse Midi-Pyrénées) using Agilent Sureprint G3 Mouse GE v2 microarrays (8x60K, design 074809) following the manufacturer's instructions. For each sample, Cyanine-3 (Cy3) labeled cRNA was prepared from 50 ng of total RNA using the One-Color Quick Amp Labeling kit (Agilent Technologies) according to the manufacturer's instructions, followed by Agencourt RNAClean XP (Agencourt Bioscience Corporation, Beverly, Massachusetts). Dye incorporation and cRNA yield were checked using DropsenseTM 96 UV/VIS droplet reader (Trinean, Belgium). 600 ng of Cy3-labelled cRNA were hybridized on the microarray slides following the manufacturer's instructions. Immediately after washing, the slides were scanned on Agilent G2505C Microarray Scanner using Agilent Scan Control A.8.5.1 software and fluorescence signal extracted using Agilent Feature Extraction software v10.10.1.1 with default parameters.

• Bioinformatics and statistics

Cohen's effect size was calculated as follows: $d = \dfrac{\bar{x}_1 - \bar{x}_2}{s}$ with $\bar{x}_1$= mean of Group 1, $\bar{x}_2$= mean of Group 2, $s$ = standard deviation

• Data editing and statistical analysis of mouse datasets

All data editing and statistical analysis have been performed thanks to R software in its 4.2.3 version, in Rstudio (2022.07.2).

For mouse microarray data, differential analysis between the three groups (R, I, P) was performed with the limma package (v3.54.2) through the lmFit() and eBayes() functions. A selection threshold was applied at 0.05 on the Benjamini-Hochberg adjusted p-values.

For mouse RNA-Seq data, the standard edgeR (v3.40.2) procedure was applied to clean and transform the data. Genes with a cpm transformed counts mean below 10 were first rejected from analysis as low expressed. Before differential analysis a TMM (trimmed mean of M values) normalisation procedure was applied on filtered raw counts to compensate technical bias at the samples level. EdgeR quasi-likelihood F-test was then used to select significant genes with a Benjamini-Hochberg adjusted p-values below a 0.05 threshold. Further analysis were applied on log(cpm) transformed and thus linearised counts data.

• Principal Component Analysis

The discriminant capacity of the genes lists obtained were tested by PCA using the R FactomineR package on various mouse and human datasets.

• Hierachical Clustering Analysis

The hclust function was used to perform hierarchical clustering (based on a Ward.D2 aggregation algorithm) in order to build a classification based on physical parameters measured at T18.

• PLS-DA

Partial Least Square discriminant analyses was used to identify the important variables (i.e., mRNA) for group classification (R package mixOmics). PLS-DA is a PLS approach in which only one dependent variable represents the class or group membership. The variable influence on projection (VIP) indicates the influence of each variable on the discrimination between the different groups. For each model, the most important transcripts in the classification were ranked according to decreasing VIP. Genes with a VIP score >1.5 were considered as contributing meaningfully to the PLS-DA model. For each PLS-DA model built, a permutation test was realized to evaluate whether the observed discrimination between groups is statistically significant or could have occurred by chance. The permutation test for PLS-DA involves randomly permuting the group membership of the observations and recalculating the PLS-DA model for each permutation. The performance of each of the model obtained are assessed by their classification error rate and compared to the error rate of the true model. This procedure is repeated 500 times.

• P-integration : Mixomics MINT-PLSDA

In order to combine the inventors' own mouse data with external datasets and to better evaluate the discriminant potential of the identified markers, an horizontal integration approach (P-integration) was implemented thanks to the R mixOmics package (v6.22.0) and its MINT functions (http://mixomics.org/mixmint). This MINT method is dedicated to the integration of multiple datasets, from multiple sources, measured on the same set of genes, thus quantified in different context (studies). Here supervised pls-da tools have been used for modelisation. Basically, 5 components models have been created. The samples have been projected in factorial plans to assess the groups separation globally and at the study level. The genes contributions to the 2 first factorial axis have also been calculated. ROC curves and their area were used as first indicator of model's quality. Finally using one by one human datasets for external validation, new ROC curves could be calculated, this time associated with error rate and balanced error rate values, adding an increased confidence in the integrative classification models.

Seven datasets have been combined : the 3 mouse datasets from the inventors' experiments with 4 human ones. All have been aligned at the features (variables) level, from the 197 genes list. This alignment kept 143 variables quantified for 141 samples corresponding to the whole cases (individuals) from the 7 integrated datasets. These samples where annotated as either « lean » or « obese » to characterise the two physiological states under study. This gives a global vector used as a target to define the classes to discriminate. A last vector defining the studies blocks was added to split the fusion dataframe into the seven experimental context and fulfill the functions options. All the external studies samples were included, with the aim of gaining robust models overwhelming potential side effects.

In MINT toolbox, the supervised framework with its mint.plsda et mint.splsda was used to build two successive classification models. The pls algorithm is especially useful in the case of a limited number of samples in comparison to the number of quantified features. A first basic model including all the common variable, without any kind of further selection, based on 5 components was produced and its performance assessed. Secondly, an optimised model was released through the sparse method, that reduced model's dimensions to a single component and the diversity of features to 109 genes from the 143 initial ones. These two models help in discriminative and predictive purposes.

• Functional enrichment analysis

Online Metascape (http://metascape.org, v3.5) allowed the functional annotation of the gene sets. Custom analysis used a specific background derived from common transcriptome between all the human and mice datasets.

• Data and code availability

- Data resources

The ArrayExpress accession number for the transcriptomic data reported in this paper are: E-MTAB-13877 for data from model 1 (diet-induced obesity) at TO, E-MTAB-13878 for data from model 1 at T1 and E-MTAB-13879 for data from model 2 (nutritional programming).

- Description of human datasets

The inventors used transcriptomic data from already published studies that are openly available in the GEO repository database.

E-GEOD-2508 dataset (Lillioja, S., Bogardus, C., 1988. Diabetes/Metabolism Reviews 4(5): 517-40, Doi: 10.1002/dmr.5610040508) consisted in microarray data obtained from isolated adipocytes of subcutaneous adipose depot of Pima Indians, male or female and lean or obese. Pima Indian are known to be a population with one of the highest prevalence rates of obesity and type 2 diabetes. These data have been obtained from a sufficiently large cohort with a minimum number of subjects of 9 for the obese male group.

E-GEOD-9624 dataset (Aguilera, C., et al., 2015. International Journal of Molecular Sciences 16(12): 7723-37, Doi: 10.3390/ijms16047723) consisted in microarray data obtained from visceral WAT of lean (n=6) and obese (n=5) prepubertal children.

MTAB-6728 dataset (Bjune, J.-I., et al., 2019. International Journal of Obesity 43(11): 2151-62, Doi: 10.1038/s41366-018-0275-y) consisted in microarray data obtained from isolated adipocytes of subcutaneous adipose depot from a large number of lean (n=12) and obese (n=12) individuals whose sex has not been specified.

GSE-166047 dataset Rey, F., et al., 2021. International Journal of Molecular Sciences 22(4): 1989, Doi: 10.3390/ijms22041989) consisted in microarray data obtained from subcutaneous adipose depot from lean (n=5) and obese (n=5) female.

**Claims**

1. Use of expression level of a gene signature comprising or consisting of GLUL, SFRP4, ITIH5, GPC1, VGLL3 and C1QTNF1 as a biomarker in a biological sample for determining a predisposition to develop obesity in a subject.

2. A method for predicting if a subject has a predisposition to develop obesity, said method comprising providing a biological sample from said subject and determining an expression level of the genes GLUL, SFRP4, ITIH5, GPC1, VGLL3 and C1QTNF1, thereby predicting whether said subject has a predisposition to develop obesity.

3. The use according to claim 1 or the method according to claim 2, wherein the under-expression of GLUL, and the over-expression of genes SFRP4, ITIH5, GPC1, VGLL3 and C1QTNF1 are indicative of a predisposition to develop obesity in said subject.

4. The use according to claim 1 or 3, wherein the gene signature further comprises 1, 2, 3, 4, 5, 6, 7, 8, 9, 10, 15, 20, 25, 30, 35, 40, 45, 50, 55, 60 or 65 additional genes selected from the group consisting of PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, ANXA2, SMPDL3A, RBBP6, EYA2, ARL4A, PALMD, EPS15, TPCN1, PRELP, FXN, AP1S1, PLCE1, LEO1, PCBP1, SNTA1, MDFIC, ANKRD39, NFIL3, METTL16, PPP1CC, SERPINF1, DIS3, STIM2, EMG1, CPT1A, and EHHADH.

**5.** The method according to claim 2 or 3, wherein the method further comprises determining the expression level of 1, 2, 3, 4, 5, 6, 7, 8, 9, 10, 15, 20, 25, 30, 35, 40, 45, 50, 55, 60 or 65 additional genes selected from the group consisting of PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, ANXA2, SMPDL3A, RBBP6, EYA2, ARL4A, PALMD, EPS15, TPCN1, PRELP, FXN, AP1S1, PLCE1, LEO1, PCBP1, SNTA1, MDFIC, ANKRD39, NFIL3, METTL16, PPP1CC, SERPINF1, DIS3, STIM2, EMG1, CPT1A, and EHHADH.

**6.** The use of claim 4 or the method of claim 5, wherein the additional genes are one of the following sets:

a) the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, and AATF;
b) the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, and NCBP1;
c) the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, and PAMR1;
d) the genes GLUL, SFRP4, ITIH5, GPC1, VGLL3, C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, and CERCAM;
e) the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, and SULF2;
f) the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, and LRRC20;
g) the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, and ANXA2;
h) the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, ANXA2, SMPDL3A, RBBP6, EYA2, ARL4A, PALMD, EPS15, TPCN1, PRELP, FXN, AP1S1, PLCE1, LEO1, PCBP1, SNTA1, MDFIC, ANKRD39, and NFIL3; or i) the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, ANXA2, SMPDL3A, RBBP6, EYA2, ARL4A, PALMD, EPS15, TPCN1, PRELP, FXN, AP1S1, PLCE1, LEO1, PCBP1, SNTA1, MDFIC, ANKRD39, NFIL3, METTL16, PPP1CC, SERPINF1, DIS3, STIM2, EMG1, CPT1A, and EHHADH.

7. The use or method according to claim 6, wherein

• In the set a), underexpression of PHB2, SFXN2, IRS1, AACS, MTMR4, AATF, SLC25A38, DNAJB9, HOXA5, NDUFS2, BAG4, and NCBP1 and overexpression of PCOLCE2, LHFPL2, TUBA1A, S100A11, ADAP2, SYNPO2, HEBP1, COL6A1, FADS3, MAN1B1, and PID1 are indicative of a predisposition to develop obesity in said subject; or

• in the set b), underexpression of PHB2, SFXN2, IRS1, AACS, MTMR4, AATF, SLC25A38, DNAJB9, HOXA5, NDUFS2, BAG4, and NCBP1 and overexpression of PCOLCE2, LHFPL2, TUBA1A, S100A11, ADAP2, SYNPO2, HEBP1, COL6A1, FADS3, MAN1B1, PID1, COL6A2, PMEPA1 , and HTRA3 are indicative of a predisposition to develop obesity in said subject; or

• in the set c), underexpression of PHB2, SFXN2, IRS1, AACS, MTMR4, AATF, SLC25A38, DNAJB9, HOXA5, NDUFS2, BAG4, NCBP1, DGAT1, and SIK3, and overexpression of PCOLCE2, LHFPL2, TUBA1A, S100A11, ADAP2, SYNPO2, HEBP1, COL6A1, FADS3, MAN1B1, PID1, COL6A2, PMEPA1, HTRA3, RNASEL, and PAMR1 are indicative of a predisposition to develop obesity in said subject; or

• in the set d), underexpression of PHB2, SFXN2, IRS1, AACS, MTMR4, AATF, SLC25A38, DNAJB9, HOXA5, NDUFS2, BAG4, NCBP1, DGAT1, SIK3, PPP1R15B, and NT5DC1 and overexpression of PCOLCE2, LHFPL2, TUBA1A, S100A11, ADAP2, SYNPO2, HEBP1, COL6A1, FADS3, MAN1B1, PID1, COL6A2, PMEPA1, HTRA3, RNASEL, PAMR1, PAM, STAT6, MAN2B1, and CERCAM are indicative of a predisposition to develop obesity in said subject; or

• in the set e), underexpression of PHB2, SFXN2, IRS1, AACS, MTMR4, AATF, SLC25A38, DNAJB9, HOXA5, NDUFS2, BAG4, NCBP1, DGAT1, SIK3, PPP1R15B, NT5DC1, SLC25A34, PPP1R1A, SLC27A1, TST, PDSS1, and LARP1 and overexpression of PCOLCE2, LHFPL2, TUBA1A, S100A11, ADAP2, SYNPO2, HEBP1, COL6A1, FADS3, MAN1B1, PID1, COL6A2, PMEPA1, HTRA3, RNASEL, PAMR1, PAM, STAT6, MAN2B1, CERCAM, ANXA1, COL18A1, ANKRD29, METRN, MAN2B2, IAH1, RTN2, FLOT1, and SULF2 are indicative of a predisposition to develop obesity in said subject; or

• in the set f), underexpression of PHB2, SFXN2, IRS1, AACS, MTMR4, AATF, SLC25A38, DNAJB9, HOXA5, NDUFS2, BAG4, NCBP1, DGAT1, SIK3, PPP1R15B, NT5DC1, SLC25A34, PPP1R1A, SLC27A1, TST, PDSS1, LARP1, CSTF1, GDE1, UBLCP1, NUP54, ATAD3A, LCMT2, and BRWD1 and overexpression of PCOLCE2, LHFPL2, TUBA1A, S100A11, ADAP2, SYNPO2, HEBP1, COL6A1, FADS3, MAN1B1, PID1, COL6A2, PMEPA1, HTRA3, RNASEL, PAMR1, PAM, STAT6, MAN2B1, CERCAM, ANXA1, COL18A1, ANKRD29, METRN, MAN2B2, IAH1, RTN2, FLOT1, SULF2, HIP1, REEPS, and LRRC20 are indicative of a predisposition to develop obesity in said subject; or

• in the set g), underexpression of PHB2, SFXN2, IRS1, AACS, MTMR4, AATF, SLC25A38, DNAJB9, HOXA5, NDUFS2, BAG4, NCBP1, DGAT1, SIK3, PPP1R15B, NT5DC1, SLC25A34, PPP1R1A, SLC27A1, TST, PDSS1, LARP1, CSTF1, GDE1, UBLCP1, NUP54, ATAD3A, LCMT2, BRWD1, LSM1, CSE1L, SLTM, JTB, HSPA9, RARRES1, and INTS4 and overexpression of PCOLCE2, LHFPL2, TUBA1A, S100A11, ADAP2, SYNPO2, HEBP1, COL6A1, FADS3, MAN1B1, PID1, COL6A2, PMEPA1, HTRA3, RNASEL, PAMR1, PAM, STAT6, MAN2B1, CERCAM, ANXA1, COL18A1, ANKRD29, METRN, MAN2B2, IAH1, RTN2, FLOT1, SULF2, HIP1, REEPS, LRRC20, PGM2L1, GPX7, MAP1LC3A, ARHGEF6, RSU1, AHNAK, SPATA6, PPFIA3, LGALS1, and ANXA2 are indicative of a predisposition to develop obesity in said subject; or

• in the set h), underexpression of PHB2, SFXN2, IRS1, AACS, MTMR4, AATF, SLC25A38, DNAJB9, HOXA5, NDUFS2, BAG4, NCBP1, DGAT1, SIK3, PPP1R15B, NT5DC1, SLC25A34, PPP1R1A, SLC27A1, TST, PDSS1, LARP1, CSTF1, GDE1, UBLCP1, NUP54, ATAD3A, LCMT2, BRWD1, LSM1, CSE1L, SLTM, JTB, HSPA9, RARRES1, INTS4, RBBP6, FXN, LEO1, PCBP1, and NFIL3 and overexpression of PCOLCE2, LHFPL2, TUBA1A, S100A11, ADAP2, SYNPO2, HEBP1, COL6A1, FADS3, MAN1B1, PID1, COL6A2, PMEPA1, HTRA3, RNASEL, PAMR1, PAM, STAT6, MAN2B1, CERCAM, ANXA1, COL18A1, ANKRD29, METRN, MAN2B2, IAH1, RTN2, FLOT1, SULF2, HIP1, REEPS, LRRC20, PGM2L1, GPX7, MAP1LC3A, ARHGEF6, RSU1, AHNAK, SPATA6, PPFIA3, LGALS1, ANXA2, SMPDL3A, EYA2, ARL4A, PALMD, EPS15, TPCN1, PRELP, AP1S1, PLCE1, SNTA1, MDFIC, and ANKRD39 are indicative of a predisposition to develop obesity in said subject; or

• in the set i), underexpression of PHB2, SFXN2, IRS1, AACS, MTMR4, AATF, SLC25A38, DNAJB9, HOXA5, NDUFS2, BAG4, NCBP1, DGAT1, SIK3, PPP1R15B, NT5DC1, SLC25A34, PPP1R1A, SLC27A1, TST, PDSS1, LARP1, CSTF1, GDE1, UBLCP1, NUP54, ATAD3A, LCMT2, BRWD1, LSM1, CSE1L, SLTM, JTB, HSPA9, RARRES1, INTS4, RBBP6, FXN, LEO1, PCBP1, NFIL3, METTL16, PPP1CC, DIS3, EMG1, and EHHADH and overexpression of PCOLCE2, LHFPL2, TUBA1A, S100A11, ADAP2, SYNPO2, HEBP1, COL6A1, FADS3, MAN1B1, PID1, COL6A2, PMEPA1, HTRA3, RNASEL, PAMR1, PAM, STAT6, MAN2B1, CERCAM, ANXA1, COL18A1, ANKRD29, METRN, MAN2B2, IAH1, RTN2, FLOT1, SULF2, HIP1, REEPS, LRRC20, PGM2L1, GPX7, MAP1LC3A, ARHGEF6, RSU1, AHNAK, SPATA6, PPFIA3, LGALS1, ANXA2, SMPDL3A, EYA2, ARL4A, PALMD, EPS15, TPCN1, PRELP, AP1S1, PLCE1, SNTA1, MDFIC, ANKRD39, SERPINF1, STIM2, and CPT1A are indicative of a predisposition to develop obesity in said subject.

8. The use according to any one of claims 1, 3, 4 and 6-7 or the method according to any one of claims 2, 3 and 5-7, wherein the biological sample comprises adipose tissue, in particular an adipose tissue biopsy sample.

9. The use of any one of claims 1, 3, 4 and 6-8 or the method according to any one of claims 2, 3 and 5-8, wherein the expression level of genes is detected and/or quantified at the mRNA level.

10. The use or method of claim 9, wherein the mRNA level is detected and/or quantified by transcription-mediated amplification, northern blot, reverse transcription polymerase chain reaction (RT-PCR), Serial Analysis of Gene Expression (SAGE), DNA microarray, tiling array, digital PCR, *in situ* hybridization, or RNA sequencing (RNA-Seq).

11. The use or method according to claim 3 or 7, wherein the expression level of genes is compared to a reference of expression level, preferably the expression level in a population of lean subjects without a predisposition to obesity.

12. Kit comprising analysis means for detecting and/or quantifying in a biological sample the expression level of the genes GLUL, SFRP4, ITIH5, GPC1, VGLL3 and C1QTNF1, the kit being suitable for detecting and/or quantifying the expression level of less than 150 genes.

13. The kit of claim 12, further comprising analysis means for detecting and/or quantifying in a sample the expression level of

• the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, and AATF;
• the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, and NCBP1;
• the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, and PAMR1;
• the genes GLUL, SFRP4, ITIH5, GPC1, VGLL3, C1QTNF1, PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, and CERCAM;
• the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, and SULF2;
• the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, and LRRC20;
• the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEPS, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, and ANXA2;

• the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEP5, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, ANXA2, SMPDL3A, RBBP6, EYA2, ARL4A, PALMD, EPS15, TPCN1, PRELP, FXN, AP1S1, PLCE1, LEO1, PCBP1, SNTA1, MDFIC, ANKRD39, and NFIL3; or

• the genes PCOLCE2, PHB2, SFXN2, LHFPL2, TUBA1A, S100A11, IRS1, ADAP2, SYNPO2, AACS, MTMR4, HEBP1, COL6A1, FADS3, MAN1B1, PID1, AATF, SLC25A38, DNAJB9, COL6A2, HOXA5, NDUFS2, BAG4, PMEPA1, HTRA3, NCBP1, DGAT1, RNASEL, SIK3, PAMR1, PAM, STAT6, PPP1R15B, NT5DC1, MAN2B1, CERCAM, ANXA1, COL18A1, SLC25A34, ANKRD29, METRN, MAN2B2, PPP1R1A, IAH1, SLC27A1, TST, RTN2, FLOT1, PDSS1, LARP1, SULF2, CSTF1, HIP1, GDE1, UBLCP1, NUP54, ATAD3A, LCMT2, BRWD1, REEP5, LRRC20, LSM1, CSE1L, SLTM, PGM2L1, GPX7, MAP1LC3A, ARHGEF6, JTB, RSU1, HSPA9, AHNAK, SPATA6, RARRES1, PPFIA3, INTS4, LGALS1, ANXA2, SMPDL3A, RBBP6, EYA2, ARL4A, PALMD, EPS15, TPCN1, PRELP, FXN, AP1S1, PLCE1, LEO1, PCBP1, SNTA1, MDFIC, ANKRD39, NFIL3, METTL16, PPP1CC, SERPINF1, DIS3, STIM2, EMG1, CPT1A, and EHHADH.

14. Use of the kit of claim according to claim 12 or 13 for determining if a subject has a predisposition to develop obesity.

15. A method for monitoring a subject, comprising predicting if a subject has a predisposition to develop obesity according to a method as defined in any one of claims 2, 3 and 5-11 and selecting the subject having said predisposition as susceptible to benefit from a preventive action against obesity.

**FIGURE 1A**

Adult
Balb/c males

Resistant (R)  Intermediate (I)  Prone (P)

HFD
18 weeks

pgWAT biopsy at T0  pgWAT at T18

**FIGURE 1B**

**FIGURE 1C**

Cluster dendrogram

☐ Resistant (R)
▨ Intermediate (I)
▨ Prone (P)

**FIGURE 1D**

**FIGURE 2A**

**FIGURE 2B**

**FIGURE 2C**

PCA 3387 VIP T0 dataset

PCA 1746 VIP T18 dataset

## FIGURE 3A

## FIGURE 3B

**FIGURE 3C**

**FIGURE 3D**

**FIGURE 3E**

**FIGURE 4A**

**FIGURE 4B**

**FIGURE 4C**

**FIGURE 5A**

T0
(3387 -> 2568 unique gene symbol)

T18
(1746 -> 1335 unique gene symbol)

1705  447  524

**201**

215  163

1294

ABD
(1889 -> 1873 unique gene symbol)

**FIGURE 5B**

PCA (197 VIP of T0 dataset)

group
- Intermediate
- Resistant
- Prone

**FIGURE 6A**

**FIGURE 6B**

ROC Curve using Comp 1

Outcome
lean vs obese: 0.812

FIGURE 6C

FIGURE 6D

ROC Curve using Comp 1

FIGURE 6E

## EUROPEAN SEARCH REPORT

**Application Number**

EP 24 30 6297

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/225047 A1 (SALONEN JUKKA T [FI]) 6 September 2012 (2012-09-06) | 12,13 | INV. C12Q1/6883 |
| A | * the whole document * * para. 24, 39, 52-53, 59, claims 87, 91 * ----- | 1-11,14, 15 | |
| A | GHOSH SUJOY ET AL: "Gene expression profiling in whole blood identifies distinct biological pathways associated with obesity", BMC MEDICAL GENOMICS, BIOMED CENTRAL LTD, LONDON UK, vol. 3, no. 1, 1 December 2010 (2010-12-01), page 56, XP021090959, ISSN: 1755-8794, DOI: 10.1186/1755-8794-3-56 * the whole document * * abstract, Table 3; p. 5, col. 2; p. 9, col. 1; p. 10, col. 2 * ----- | 1-15 | |
| A | EP 2 921 562 A1 (UNIV TOKYO [JP]; SEKISUI MEDICAL CO LTD [JP]) 23 September 2015 (2015-09-23) * the whole document * * para. 11, 12, claim 1 * ----- | 1-15 | **TECHNICAL FIELDS SEARCHED (IPC)** C12Q |
| A | YUNJUNG KIM ET AL: "DNA microarrays to define and search for genes associated with obesity", BIOTECHNOLOGY JOURNAL, WILEY-VCH VERLAG, WEINHEIM, DE, vol. 5, no. 1, 18 December 2009 (2009-12-18), pages 99-112, XP072399618, ISSN: 1860-6768, DOI: 10.1002/BIOT.200900228 * the whole document * * abstract, Table 1 * ----- | 1-15 | |

The present search report has been drawn up for all claims

| Place of search | Date of completion of the search | Examiner |
|---|---|---|
| Munich | 7 January 2025 | Sauer, Tincuta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

**EP 24 30 6297**

This annex lists the patent family members relating to the patent documents cited in the above-mentioned European search report.
The members are as contained in the European Patent Office EDP file on
The European Patent Office is in no way liable for these particulars which are merely given for the purpose of information.

**07-01-2025**

| Patent document cited in search report | | | Publication date | Patent family member(s) | | Publication date |
|---|---|---|---|---|---|---|
| US 2012225047 | A1 | | 06-09-2012 | EP 2501826 | A1 | 26-09-2012 |
| | | | | US 2012225047 | A1 | 06-09-2012 |
| | | | | WO 2011058232 | A1 | 19-05-2011 |
| EP 2921562 | A1 | | 23-09-2015 | EP 2578699 | A1 | 10-04-2013 |
| | | | | EP 2921562 | A1 | 23-09-2015 |
| | | | | JP 5422799 | B2 | 19-02-2014 |
| | | | | JP 2013252136 | A | 19-12-2013 |
| | | | | JP WO2011149057 | A1 | 25-07-2013 |
| | | | | US 2013143748 | A1 | 06-06-2013 |
| | | | | WO 2011149057 | A1 | 01-12-2011 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011058232 A
- WO 2005021787 A
- WO 2003097683 A

**Non-patent literature cited in the description**

- **LILLIOJA, S.** ; **BOGARDUS, C.** *Diabetes/Metabolism Reviews*, 1988, vol. 4 (5), 517-40
- **BJUNE, J.-I. et al.** *International Journal of Obesity*, 2019, vol. 43 (11), 2151-62
- **REY, F. et al.** *International Journal of Molecular Sciences*, 2021, vol. 22 (4), 1989
- **AGUILERA, C et al.** *International Journal of Molecular Sciences*, 2015, vol. 16 (12), 7723-37
- **LILLIOJA, S.** ; **BOGARDUS, C**. *Diabetes/Metabolism Reviews*, 1988, vol. 4 (5), 517-40
- **AGUILERA, C. et al.** *International Journal of Molecular Sciences*, 2015, vol. 16 (12), 7723-37
- **REY, F et al.** *International Journal of Molecular Sciences*, 2021, vol. 22 (4), 1989